# EUROPEAN PATENT APPLICATION

(11) **EP 4 477 752 A1**
(43) Date of publication of application: **18.12.2024**
(21) Application number: 23752935.9
(22) Date of filing: 09.02.2023
(51) Int. Cl.: C12N 15/13, A61K 39/395, A61K 47/68, A61P 29/00, A61P 35/00, A61P 35/02, A61P 37/02, C07K 16/28, C07K 16/46, C12N 1/15, C12N 1/19, C12N 1/21, C12N 5/10, C12N 5/20, C12P 21/08, G01N 33/53

(54) **ANTIBODY OR FRAGMENT THEREOF THAT BINDS TO FCRL1**

(30) Priority: 09.02.2022 JP 2022019051
(71) Applicant: National Institutes of Biomedical Innovation, Health and Nutrition, Ibaraki-shi, Osaka 567-0085 (JP); Kyowa Kirin Co., Ltd., Tokyo 100-0004 (JP)
(72) Inventor: NAGATA Satoshi, Ibaraki-shi, Osaka 567-0085 (JP); ISE Tomoko, Ibaraki-shi, Osaka 567-0085 (JP); KAMADA Haruhiko, Ibaraki-shi, Osaka 567-0085 (JP); SATO Hidetaka, Tokyo 100-0004 (JP); TOKUNAGA Akihiro, Tokyo 100-0004 (JP); MATSUBARA Masahiro, Tokyo 100-0004 (JP); NAMISAKI Hiroshi, Tokyo 100-0004 (JP); YAMADA Takenao, Tokyo 100-0004 (JP)
(74) Representative: Hoffmann Eitle
(86) International application number: PCT/JP2023/004346
(87) International publication number: WO 2023/153471

(57) **Abstract**

The present invention relates to a monoclonal antibody or an antibody fragment thereof that binds to an extracellular region of FCRL1, a hybridoma that produces the antibody, a nucleic acid comprising a nucleotide sequence that encodes the antibody or the antibody fragment thereof, a cell transformed with a vector comprising the nucleic acid, a method for producing the antibody or the antibody fragment thereof using the hybridoma or the transformed cell, an antibody-drug conjugate comprising the antibody or the antibody fragment thereof, a therapeutic agent and a diagnostic agent, each of which comprises the antibody or the antibody fragment thereof, and a therapeutic method and a diagnostic method for a disease associated with FCRL1 using the antibody, the antibody fragment thereof, or the antibody-drug conjugate comprising the antibody or the antibody fragment thereof.

## Description

### TECHNICAL FIELD

The present invention relates to a monoclonal antibody or an antibody fragment thereof that binds to an extracellular region of Fc receptor-like protein 1, a hybridoma that produces the antibody, a nucleic acid comprising a nucleotide sequence that encodes the antibody or the antibody fragment thereof, a transformed cell obtained by introducing a vector comprising the nucleic acid into a host cell, a method for producing the antibody or the antibody fragment thereof using the hybridoma or the transformed cell, an antibody-drug conjugate comprising the antibody or the antibody fragment thereof, a therapeutic agent and a diagnostic agent, each of which comprises the antibody or the antibody fragment thereof, and a therapeutic method and a diagnostic method for a disease associated with Fc receptor-like protein 1 using the antibody, the antibody fragment thereof, or the antibody-drug conjugate comprising the antibody or the antibody fragment thereof.

### BACKGROUND ART

Fc receptor-like protein 1 (hereinafter, may be referred to as FCRL 1) is a membrane protein belonging to an immunoglobulin superfamily, also known by other names such as CD307a, FCRH1, IFGP1, and IRTA5. The amino acid sequence of human FCRL1 is identified in 2001 (Non-Patent Literature 1).

FCRL1 is a type I transmembrane protein expressed in B cells. FCRL1 is a protein that has three extracellular immunoglobulin-like domains, two intracellular immunoreceptor tyrosine activation motifs, and a transmembrane region (Non-Patent Literature 1). No endogenous ligand for FCRL 1 has been identified to date.

It has been reported that FCRL1 is expressed in, in addition to normal B cells, cancer cells of chronic lymphocytic leukemia, follicular lymphoma, hairy cell leukemia, and mantle cell lymphoma (Non-Patent Literatures 2 and 3). Further, it has been reported that, in recent years, FCRL 1 contributes to cancer growth (Non-Patent Literature 4).

E3, E9 (Non-Patent Literature 2), 2G5, 7G8, 5A2 (Patent Literature 1), 1F9, 2A10 (Patent Literature 2), and 5A3 (Patent Literature 3) are known as a monoclonal antibody against FCRL1. It is known that binding an immunotoxin to an anti-FCRL 1 antibody exerts cellular cytotoxicity against a cancer cell line (Non-Patent Literature 4).

### CITATION LIST

### PATENT LITERATURE

Patent Literature 1: WO2005/097185
Patent Literature 2: US Patent Application Publication No. 2006/0216232
Patent Literature 3: WO2006/037048

### NON-PATENT LITERATURE

Non-Patent Literature 1: Davis RS., et al. "Identification of a family of Fc receptor homologs with preferential B cell expression" Proceedings of the National Academy of Sciences of the United States of America 98. 17 (2001): 9772-9777.
Non-Patent Literature 2: Du X., et al. "FCRL 1 on chronic lymphocytic leukemia, hairy cell leukemia, and B-cell non-Hodgkin lymphoma as a target of immunotoxins" Blood 111. 1 (2008): 338-343.
Non-Patent Literature 3: Auat M., et al. "Evaluation of CD307a expression patterns during normal B-cell maturation and in B-cell malignancies by flow cytometry" Cytometry Part B: Clinical Cytometry 94. 4 (2018): 588-595.
Non-Patent Literature 4: Yousefi Z., et al. "Fc Receptor-Like 1 as a Promising Target for Immunotherapeutic Interventions of B-Cell-Related Disorders" Biomarker Insights 14 (2018): 1-9.

### SUMMARY OF INVENTION

### TECHNICAL PROBLEM

An object of the present invention is to provide a novel monoclonal antibody or an antibody fragment thereof that binds to an extracellular region of FCRL 1, a hybridoma that produces the antibody, a nucleic acid comprising a nucleotide sequence that encodes the antibody or the antibody fragment thereof, a transformed cell obtained by introducing a vector comprising the nucleic acid into a host cell, a method for producing the antibody or the antibody fragment thereof using the hybridoma or the transformed cell, an antibody-drug conjugate comprising the antibody or the antibody fragment thereof, a therapeutic agent and a diagnostic agent, each of which comprises the antibody or the antibody fragment thereof, and a therapeutic method and a diagnostic method for a disease associated with FCRL1 using the antibody, the antibody fragment thereof, or the antibody-drug conjugate comprising the antibody or the antibody fragment thereof.

### SOLUTION TO PROBLEM

The present invention relates to the following 1 to 26.
1. An antibody or an antibody fragment thereof, which is a monoclonal antibody or an antibody fragment thereof that binds to Fc receptor-like protein 1 (hereinafter, abbreviated as FCRL 1), in which
   the antibody is any one antibody selected from the following (a) to (g):
   (a) an antibody in which complementarity determining regions (hereinafter, abbreviated as CDR) 1 to 3 of a heavy chain variable region (hereinafter, abbreviated as VH) comprise the amino acid sequences represented by SEQ ID NOs: 20 to 22, respectively, and CDRs 1 to 3 of a light chain variable region (hereinafter, abbreviated as VL) comprise the amino acid sequences represented by SEQ ID NOs: 24 to 26, respectively,
   (b) an antibody in which CDRs 1 to 3 of a VH comprise the amino acid sequences represented by SEQ ID NOs: 28 to 30, respectively, and CDRs 1 to 3 of a VL comprise the amino acid sequences represented by SEQ ID NOs: 32 to 34, respectively,
   (c) an antibody in which CDRs 1 to 3 of a VH comprise the amino acid sequences represented by SEQ ID NOs: 36 to 38, respectively, and CDRs 1 to 3 of a VL comprise the amino acid sequences represented by SEQ ID NOs: 40 to 42, respectively,
   (d) an antibody in which CDRs 1 to 3 of a VH comprise the amino acid sequences represented by SEQ ID NOs: 44 to 46, respectively, and CDRs 1 to 3 of a VL comprise the amino acid sequences represented by SEQ ID NOs: 48 to 50, respectively,
   (e) an antibody in which CDRs 1 to 3 of a VH comprise the amino acid sequences represented by SEQ ID NOs: 52 to 54, respectively, and CDRs 1 to 3 of a VL comprise the amino acid sequences represented by SEQ ID NOs: 56 to 58, respectively, and
   (f) an antibody in which CDRs 1 to 3 of a VH comprise the amino acid sequences represented by SEQ ID NOs: 60 to 62, respectively, and CDRs 1 to 3 of a VL comprise the amino acid sequences represented by SEQ ID NOs: 64 to 66, respectively, and
   (g) an antibody in which CDRs 1 to 3 of a VH comprise the amino acid sequences represented by SEQ ID NOs: 36 to 38, respectively, and CDRs 1 to 3 of a VL comprise the amino acid sequences represented by SEQ ID NOs: 40, 71, and 42, respectively.
2. An antibody or an antibody fragment thereof, which is a monoclonal antibody or an antibody fragment thereof that binds to FCRL 1, in which
   the antibody is any one antibody selected from the following (2b-1) to (2b-4), (2c-1), (2c-2), and (2g-1):
   (2b-1) an antibody in which a VH comprises the amino acid sequence represented by SEQ ID NO: 72 and a VL comprises the amino acid sequence represented by SEQ ID NO: 68,
   (2b-2) an antibody in which a VH comprises the amino acid sequence represented by SEQ ID NO: 73 and a VL comprises the amino acid sequence represented by SEQ ID NO: 74,
   (2b-3) an antibody in which a VH comprises the amino acid sequence represented by SEQ ID NO: 72 and a VL comprises the amino acid sequence represented by SEQ ID NO: 74,
   (2b-4) an antibody in which a VH comprises the amino acid sequence represented by SEQ ID NO: 73 and a VL comprises the amino acid sequence represented by SEQ ID NO: 68,
   (2c-1) an antibody in which a VH comprises the amino acid sequence represented by SEQ ID NO: 75 and a VL comprises the amino acid sequence represented by SEQ ID NO: 76,
   (2c-2) an antibody in which a VH comprises the amino acid sequence represented by SEQ ID NO: 77 and a VL comprises the amino acid sequence represented by SEQ ID NO: 76, and
   (2g-1) an antibody in which a VH comprises the amino acid sequence represented by SEQ ID NO: 77 and a VL comprises the amino acid sequence represented by SEQ ID NO: 78.
3. The antibody or the antibody fragment thereof according to the above 1 or 2, in which
   a heavy chain constant region of the antibody is a heavy chain constant region of IgG.
4. The antibody or the antibody fragment thereof according to the above 3, in which
   the heavy chain constant region of the antibody comprises the amino acid sequence represented by SEQ ID NO: 79 or 80.
5. The antibody or the antibody fragment thereof according to any one of the above 1 to 4, in which
   the antibody is a recombinant antibody.
6. The antibody or the antibody fragment thereof according to the above 5, in which
   the recombinant antibody is one selected from the group consisting of a chimeric antibody, a humanized antibody, and a human antibody.
7. The antibody fragment according to any one of the above 1 to 6, in which
   the antibody fragment is one selected from Fab, Fab', F(ab')₂, a single chain antibody (scFv), a dimerized V region (diabody), a disulfide-stabilized V region (dsFv), and a peptide comprising a CDR.
8. A hybridoma that produces the antibody according to any one of the above 1 to 6.
9. A nucleic acid comprising a nucleotide sequence that encodes the antibody or the antibody fragment thereof according to any one of the above 1 to 7.
10. A vector comprising the nucleic acid according to the above 9.
11. A transformed cell obtained by introducing the vector according to the above 10 into a host cell.
12. A method for producing the antibody or the antibody fragment thereof according to any one of the above 1 to 7, including:
   culturing the hybridoma according to the above 8 or the transformed cell according to the above 11 in a medium, and collecting the antibody or the antibody fragment thereof from a culture.
13. An antibody-drug conjugate containing:
   the antibody or the antibody fragment thereof according to any one of the above 1 to 7.
14. The antibody-drug conjugate according to the above 13, in which
   the antibody-drug conjugate contains the antibody or the antibody fragment thereof linked to a drug via a linker
15. A composition containing:
   the antibody or the antibody fragment thereof according to any one of the above 1 to 7, or the antibody-drug conjugate according to the above 13 or 14.
16. A reagent for detecting or measuring FCRL 1, containing:
   the antibody or the antibody fragment thereof according to any one of the above 1 to 7, or the antibody-drug conjugate according to the above 13 or 14.
17. A diagnostic agent for a disease associated with FCRL 1, containing:
   the antibody or the antibody fragment thereof according to any one of the above 1 to 7, or the antibody-drug conjugate according to the above 13 or 14.
18. The diagnostic agent according to the above 17, in which
   the disease associated with FCRL1 is cancer, an autoimmune disease, or an inflammatory disease.
19. A therapeutic agent for a disease associated with FCRL 1, containing:
   the antibody or the antibody fragment thereof according to any one of the above 1 to 7, or the antibody-drug conjugate according to the above 13 or 14.
20. The therapeutic agent according to the above 19, in which
   the disease associated with FCRL1 is cancer, an autoimmune disease, or an inflammatory disease.
21. A diagnostic method for a disease associated with FCRL 1, including:
   using the antibody or the antibody fragment thereof according to any one of the above 1 to 7, or the antibody-drug conjugate according to the above 13 or 14.
22. A therapeutic method for a disease associated with FCRL 1, including:
   administering the antibody or the antibody fragment thereof according to any one of the above 1 to 7, or the antibody-drug conjugate according to the above 13 or 14.
23. Use of the antibody or the antibody fragment thereof according to any one of the above 1 to 7, or the antibody-drug conjugate according to the above 13 or 14, for manufacturing a diagnostic agent for a disease associated with FCRL 1.
24. Use of the antibody or the antibody fragment thereof according to any one of the above 1 to 7, or the antibody-drug conjugate according to the above 13 or 14, for manufacturing a therapeutic agent for a disease associated with FCRL 1.
25. Use of the antibody or the antibody fragment thereof according to any one of the above 1 to 7, or the antibody-drug conjugate according to the above 13 or 14, for use as a diagnostic agent for a disease associated with FCRL1.
26. Use of the antibody or the antibody fragment thereof according to any one of the above 1 to 7, or the antibody-drug conjugate according to the above 13 or 14, for use as a therapeutic agent for a disease associated with FCRL1.

### ADVANTAGEOUS EFFECTS OF INVENTION

The monoclonal antibody or the antibody fragment thereof of the present invention selectively binds to an extracellular region of human FCRL1. In particular, the monoclonal antibody or the antibody fragment thereof of the present invention exhibits an excellent effect when used in an antibody-drug conjugate (hereinafter, also referred to as ADC), as compared with an existing FCRL1 antibody. Therefore, the monoclonal antibody or the antibody fragment thereof of the present invention can be used as a therapeutic agent or a diagnostic agent for a disease associated with human FCRL1.

### BRIEF DESCRIPTION OF DRAWINGS

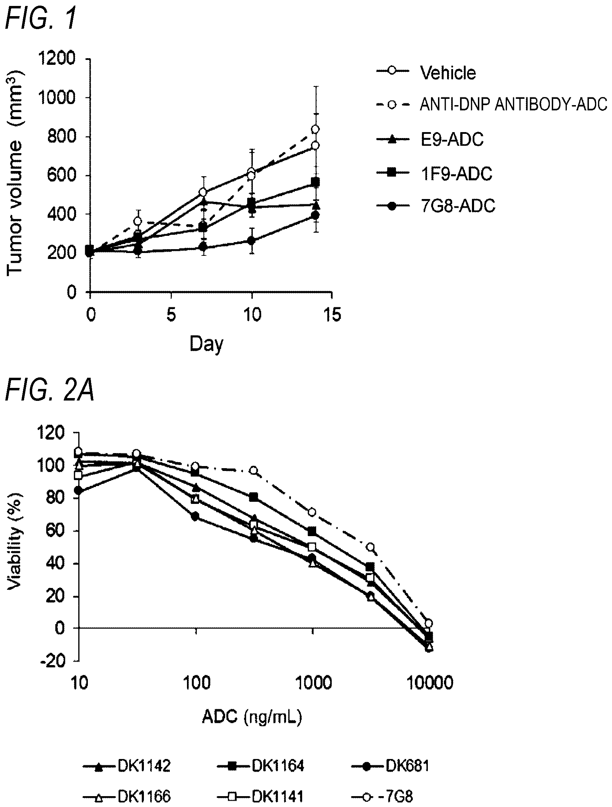

[FIG. 1] FIG. 1 shows a result of measuring an anti-tumor effect of an antibody-drug conjugate, in which a payload linker SG3249 is linked to a known anti-human FCRL1 antibody, on an SU-DHL-6 cell subcutaneous grafted mouse model, in which a vertical axis in FIG. 1 indicates a tumor size (mm³), a horizontal axis in FIG. 1 indicates the number of days after administration of ADC to the SU-DHL-6 cell subcutaneous grafted mouse model, E9, 1F9, and 7G8 are used as the known anti-human FCRL1 antibody, and an anti-2,4-dinitrophenol (DNP) IgG1 antibody is used as a negative antibody.
[FIG. 2A] FIG. 2A shows a result of measuring an effect of an ADC, in which a payload linker SG3249 is linked to a novel anti-human FCRL 1 antibody, on survival of SU-DHL-6 cells, in which a vertical axis in FIG. 2A indicates cell viability (%), and the number of cells under a condition of not treating ADC is 100%, a horizontal axis in FIG. 2A indicates a concentration of ADC added to SU-DHL-6 cells, DK1142, DK1164, DK681, DK1166, and DK1141 are used as the novel anti-human FCRL1 antibody, and 7G8 is used as the known anti-human FCRL1 antibody.

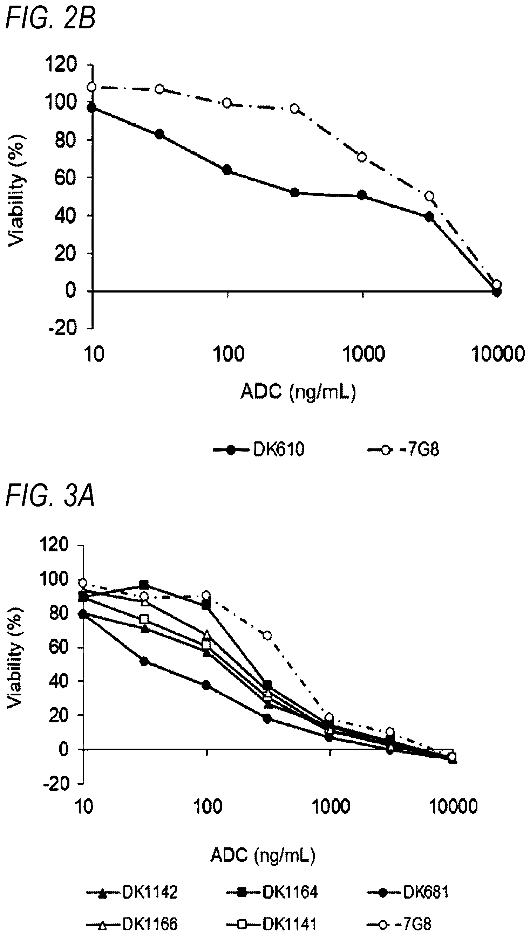

[FIG. 2B] FIG. 2B shows a result of using DK610 as the novel anti-human FCRL1 antibody in a measurement same as that in FIG. 2A.
[FIG. 3A] FIG. 3A shows a result of measuring an effect of an ADC, in which a payload linker SG3249 is linked to a novel anti-human FCRL 1 antibody, on survival of Ramos cells, in which a vertical axis in FIG. 3A indicates cell viability (%), and the number of cells under a condition of not treating ADC is 100%, a horizontal axis in FIG. 3A indicates a concentration of ADC added to Ramos cells, DK1142, DK1164, DK681, DK1166, and DK1141 are used as the novel anti-human FCRL1 antibody, and 7G8 is used as the known anti-human FCRL1 antibody.

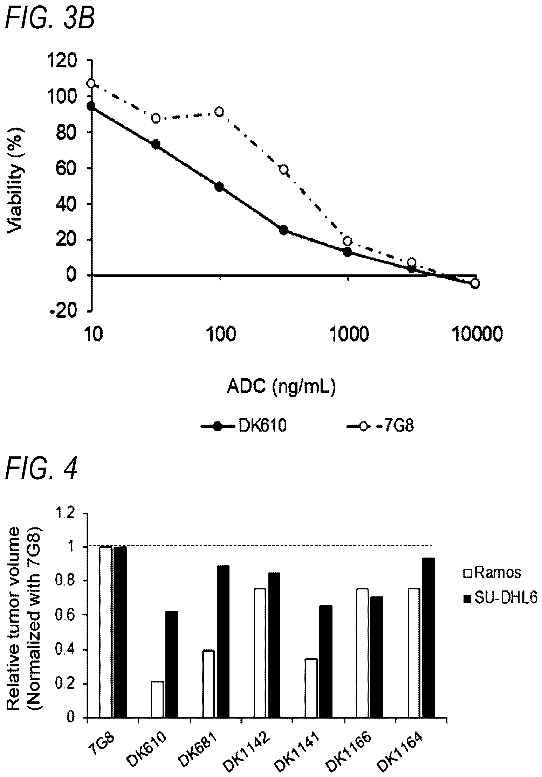

[FIG. 3B] FIG. 3B shows a result of using DK610 as the novel anti-human FCRL1 antibody in a measurement same as that in FIG. 3A.
[FIG. 4] FIG. 4 shows a result of measuring an anti-tumor effect of an ADC, in which a payload linker SG3249 is linked to a novel anti-human FCRL1 antibody, on an SU-DHL-6 cell subcutaneous grafted mouse model and a Ramos cell subcutaneous grafted mouse model, in which results on day 10 after drug administration are shown, a vertical axis in FIG. 4 indicates a relative tumor size when the tumor size of the mouse administered with 7G8 is defined as 1, DK1142, DK1164, DK681, DK1166, DK1141, and DK610 are used as the novel anti-human FCRL1 antibody, and 7G8 is used as the known anti-human FCRL 1 antibody.

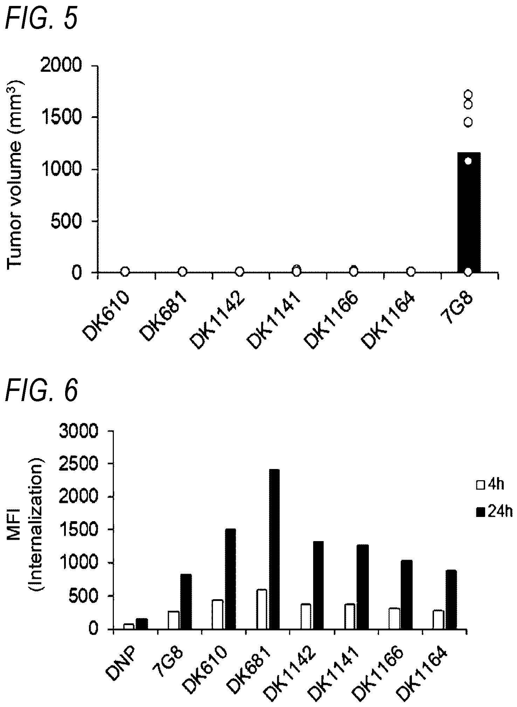

[FIG. 5] FIG. 5 shows a result of measuring an anti-tumor effect of an ADC, in which a payload linker SG3249 is linked to a novel anti-human FCRL1 antibody, on a Ramos cell subcutaneous grafted mouse model, in which results on day 42 after drug administration are shown, a vertical axis in FIG. 5 indicates a tumor size (mm³), DK1142, DK1164, DK681, DK1166, DK1141, and DK610 are used as the novel anti-human FCRL1 antibody, and 7G8 is used as the known anti-human FCRL1 antibody.
[FIG. 6] FIG. 6 shows a result of measuring antibody internalization of the novel anti-human FCRL 1 antibody in Ramos cells, in which a vertical axis in FIG. 6 indicates a fluorescence intensity, DK1142, DK1164, DK681, DK1166, DK1141, and DK610 are used as the novel anti-human FCRL1 antibody, and 7G8 is used as the known anti-human FCRL1 antibody.

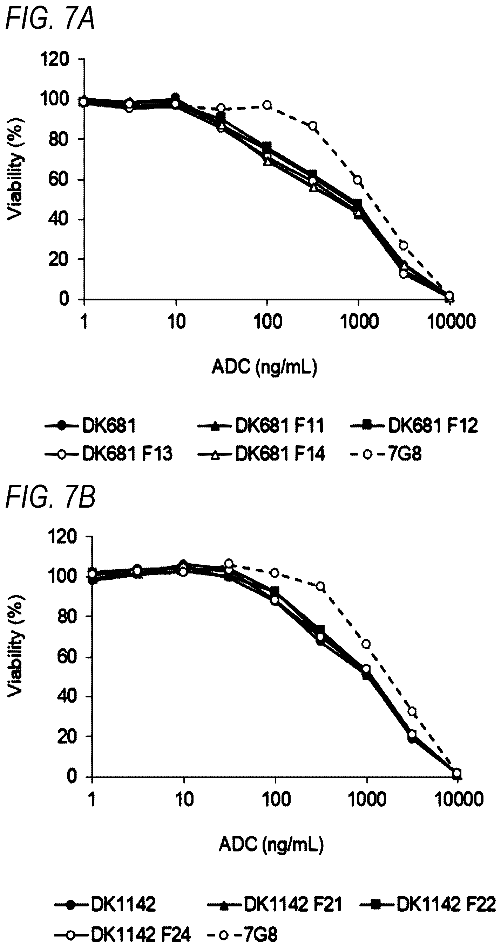

[FIG. 7A] FIG. 7A shows a result of measuring an effect of an ADC, in which a payload linker SG3249 is linked to a novel anti-human FCRL 1 antibody, on survival of SU-DHL-6 cells, in which a vertical axis in FIG. 7A indicates cell viability (%), and the number of cells under a condition of not treating ADC is 100%, a horizontal axis in FIG. 7A indicates a concentration of ADC added to SU-DHL-6 cells, DK681 is used as a novel anti-FCRL 1 chimeric antibody, and DK681 F11, DK681 F12, DK681 F13, and DK681 F14 are used as a novel anti-FCRL1 humanized antibody, and 7G8 is used as the known anti-human FCRL1 antibody.
[FIG. 7B] FIG. 7B shows a result of using DK1142 as a novel anti-FCRL 1 chimeric antibody and DK1142 F21, DK1142 F22, and DK1142 F24 as the novel anti-FCRL1 humanized antibody in a measurement same as that in FIG. 7A.

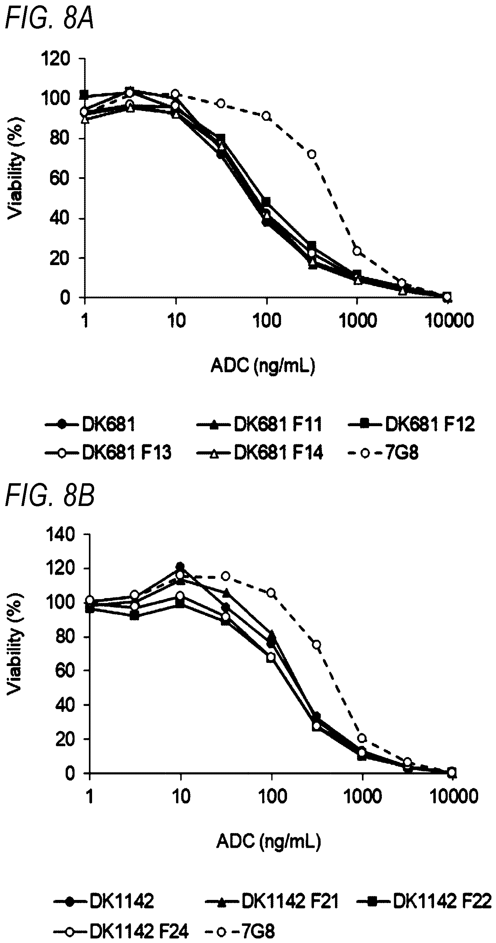

[FIG. 8A] FIG. 8A shows a result of measuring an effect of an ADC, in which a payload linker SG3249 is linked to a novel anti-human FCRL 1 antibody, on survival of Ramos cells, in which a vertical axis in FIG. 8A indicates cell viability (%), and the number of cells under a condition of not treating ADC is 100%, a horizontal axis in FIG. 8A indicates a concentration of ADC added to Ramos cells, DK681 is used as a novel anti-FCRL1 chimeric antibody, and DK681 F11, DK681 F12, DK681 F13, and DK681 F14 are used as a novel anti-FCRL1 humanized antibody, and 7G8 is used as the known anti-human FCRL 1 antibody.
[FIG. 8B] FIG. 8B shows a result of using DK1142 as a novel anti-FCRL 1 chimeric antibody and DK1142 F21, DK1142 F22, and DK1142 F24 as the novel anti-FCRL1 humanized antibody in a measurement same as that in FIG. 8A.

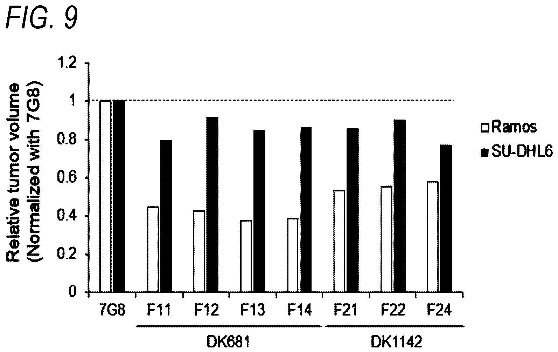

[FIG. 9] FIG. 9 shows a result of measuring an anti-tumor effect of an ADC, in which a payload linker SG3249 is linked to a novel anti-human FCRL1 antibody, on an SU-DHL-6 cell subcutaneous grafted mouse model and a Ramos cell subcutaneous grafted mouse model, in which results on day 7 after drug administration are shown, a vertical axis in FIG. 9 indicates a relative tumor size when the tumor size of the mouse administered with 7G8 is defined as 1, DK681 F11, DK681 F12, DK681 F13, DK681 F14, DK1142 F21, DK1142 F22, and DK1142 F24 are used as the novel anti-human FCRL1 antibody, and 7G8 is used as the known anti-human FCRL1 antibody.

### DESCRIPTION OF EMBODIMENTS

The present invention relates to a monoclonal antibody or an antibody fragment thereof that binds to human FCRL1.

FCRL1 is also referred to as CD307a, FCRH1, IFGP1, and IRTA5. FCRL1 belongs to an immunoglobulin superfamily and is a type 1 membrane protein consisting of 413 amino acids.

FCRL1 has two intracellular immunoreceptor tyrosine activation motifs (ITAMs). Therefore, it is expected that an activation signal is transmitted into a cell by binding of a ligand, but an endogenous ligand of FCRL 1 is not identified at the present time, and a function of FCRL 1 is not clear. It has been reported that in an experiment using a cancer cell line in recent years, FCRL 1 is associated with growth of cancer cells by controlling expression of apoptosis-related molecules.

In the present invention, examples of the human FCRL 1 include a polypeptide comprising the amino acid sequence represented by SEQ ID NO: 3 or the amino acid sequence represented by NCBI Accession Number: NP_443170, a polypeptide consisting of an amino acid sequence in which one or more amino acids are deleted, substituted, or added in the amino acid sequence represented by SEQ ID NO: 3 or the amino acid sequence represented by NCBI Accession Number: NP_443170 and having a function of the human FCRL1, and a polypeptide consisting of an amino acid sequence having a similarity of 60% or more, preferably 80% or more, further preferably 90% or more, and most preferably 95% or more to the amino acid sequence represented by SEQ ID NO: 3 or the amino acid sequence represented by NCBI Accession Number: NP_443170 and having a function of the human FCRL 1.

The polypeptide comprising an amino acid sequence in which one or more amino acids are deleted, substituted, or added in the amino acid sequence represented by SEQ ID NO: 3 or the amino acid sequence represented by NCBI Accession Number: NP_443170 can be obtained by introducing a site-specific mutagenesis into a DNA encoding the polypeptide comprising the amino acid sequence represented by SEQ ID NO: 3, for example, using a site-specific mutagenesis method [Molecular Cloning, A Laboratory Manual, Second Edition, Cold Spring Harbor Laboratory Press (1989), Current Protocols in Molecular Biology, John Wiley & Sons (1987-1997), Nucleic acids Research, 10, 6487 (1982), Proc. Natl. Acad. Sci. USA, 79, 6409 (1982), Gene, 34, 315 (1985), Nucleic Acids Research, 13, 4431 (1985), Proc. Natl. Acad. Sci. USA, 82, 488 (1985)].

The number of amino acids to be deleted, substituted, or added is not particularly limited, and is preferably 1 to several tens of amino acids, for example 1 to 20 amino acids, and more preferably 1 to several amino acids, for example 1 to 5 amino acids.

Examples of a gene encoding the human FCRL1 include the nucleotide sequence represented by SEQ ID NO: 1 and the nucleotide sequence represented by NCBI Accession Number: NM_052938. The gene encoding the human FCRL1 of the present invention also includes a gene comprising a DNA consisting of a nucleotide sequence in which one or more bases are deleted, substituted, or added in the nucleotide sequence represented by SEQ ID NO: 1 or the nucleotide sequence represented by NM_052938 and encoding a polypeptide having the function of the human FCRL1, a gene comprising a DNA consisting of a nucleotide sequence having a similarity of at least 60% to the nucleotide sequence represented by SEQ ID NO: 1 or the nucleotide sequence represented by NM_052938, preferably consisting of a nucleotide sequence having a similarity of 80% or more, further preferably consisting of a nucleotide sequence having a similarity of 95% or more and encoding a polypeptide having the function of the human FCRL1, or a gene consisting of a DNA that hybridizes under stringent conditions with a DNA comprising the nucleotide sequence represented by SEQ ID NO: 1 or the nucleotide sequence represented by NM_052938 and encoding a polypeptide having the function of the human FCRL 1.

The DNA that hybridizes under stringent conditions refers to a hybridizable DNA obtained by a colony hybridization method, a plaque hybridization method, a Southern blot hybridization method, a DNA microarray method, or the like using a DNA comprising the nucleotide sequence represented by SEQ ID NO: 1 or the nucleotide sequence represented by NM_052938 as a probe.

Specific examples thereof include a DNA that can be identified by performing hybridization [Molecular Cloning, A Laboratory Manual, Second Edition, Cold Spring Harbor Laboratory Press (1989), Current Protocols in Molecular Biology, John Wiley & Sons (1987-1997), DNA Cloning 1: Core Techniques, A Practical Approach, Second Edition, Oxford University (1995)] at 65°C in the presence of 0.7 mol/L to 1.0 mol/L sodium chloride using a filter or a slide glass on which a DNA derived from a hybridized colony or plaque, or a PCR product or an oligo DNA having the sequence is immobilized, and then washing the filter or the slide glass under a condition of 65°C using a 0.1x to 2x SSC solution (a composition of the 1x SSC solution consists of 150 mmol/L sodium chloride and 15 mmol/L sodium citrate).

Examples of the hybridizable DNA include a DNA having a similarity of at least 60% to the nucleotide sequence represented by SEQ ID NO: 1 or the nucleotide sequence represented by NM_052938, preferably include a DNA having a similarity of 80% or more, and further preferably include a DNA having a similarity of 95% or more.

Genetic polymorphisms are often observed in the nucleotide sequences of genes encoding eukaryotic proteins. The gene encoding the human FCRL1 in the present invention also includes genes used in the present invention that have small-scale mutations in nucleotide sequences thereof due to such polymorphisms.

Examples of the antibody of the present invention include an antibody that binds to both human FCRL 1 and monkey FCRL 1.

In the present invention, examples of the monkey FCRL1 include a polypeptide comprising the amino acid sequence represented by SEQ ID NO: 4 or the amino acid sequence represented by NCBI Accession Number: XP_015310712, a polypeptide consisting of an amino acid sequence in which one or more amino acids are deleted, substituted, or added in the amino acid sequence represented by SEQ ID NO: 4 or the amino acid sequence represented by NCBI Accession Number: XP_015310712 and having a function of the monkey FCRL1, and a polypeptide consisting of an amino acid sequence having a similarity of 60% or more, preferably 80% or more, further preferably 90% or more, and most preferably 95% or more to the amino acid sequence represented by SEQ ID NO: 4 or the amino acid sequence represented by NCBI Accession Number. XP_015310712 and having a function of the monkey FCRL1.

The polypeptide comprising an amino acid sequence in which one or more amino acids are deleted, substituted, or added in the amino acid sequence represented by SEQ ID NO: 4 or the amino acid sequence represented by NCBI Accession Number: XP_015310712 can be obtained, for example, by introducing a site-specific mutagenesis into a DNA encoding a polypeptide comprising the amino acid sequence represented by SEQ ID NO: 4, using a site-specific mutagenesis method or the like.

The number of amino acids to be deleted, substituted, or added is not particularly limited, and is preferably 1 to several tens of amino acids, for example 1 to 20 amino acids, and more preferably 1 to several amino acids, for example 1 to 5 amino acids.

Examples of a gene encoding the monkey FCRL1 include the nucleotide sequence represented by SEQ ID NO: 2 and the nucleotide sequence represented by NCBI Accession Number: XM_005541349. The gene encoding the monkey FCRL1 of the present invention also includes a gene comprising a DNA consisting of a nucleotide sequence in which one or more bases are deleted, substituted, or added in the nucleotide sequence represented by SEQ ID NO: 2 or the nucleotide sequence represented by XM_005541349 and encoding a polypeptide having the function of the monkey FCRL 1, a gene comprising a DNA consisting of a nucleotide sequence having a similarity of at least 60% to the nucleotide sequence represented by SEQ ID NO: 2 or the nucleotide sequence represented by XM_005541349, preferably consisting of a nucleotide sequence having a similarity of 80% or more, further preferably consisting of a nucleotide sequence having a similarity of 95% or more and encoding a polypeptide having the function of the monkey FCRL1, or a gene consisting of a DNA that hybridizes under stringent conditions with a DNA comprising the nucleotide sequence represented by SEQ ID NO: 2 or the nucleotide sequence represented by XM_005541349 and encoding a polypeptide having the function of the monkey FCRL1.

The similarity of amino acid sequences or nucleotide sequences in the present invention refers to a numerical value calculated under specific conditions by comparing two amino acid sequences or nucleotide sequences. Specifically, the similarity can be obtained by obtaining an alignment of two sequences and calculating a proportion of identical or similar residue pairs in the alignment. Algorithms such as a Needleman-Wunsch method, a Smith-Waterman method, a FASTA method, and a BLAST method are used to obtain the alignment. Examples of parameters used in each algorithm include a similarity evaluation index for a residual pair unit (in the case of amino acid sequences, for example, a substitution matrix such as BLOSUM62, BLOSUM50, and PAM30 is used, and in the case of nucleotide sequences, for example, match reward or mismatch penalty is used), and a quantitative evaluation index for a gap portion (for example, an affine gap cost function). As an example of the similarity of amino acid sequences or nucleotide sequences in the present invention, values of identities or positives that are output in association with alignment acquired by NCBI BLAST, which is a representative implementation of the BLAST method, using default parameters can be cited.

The binding of the antibody of the present invention to the extracellular region of the human FCRL 1 can be confirmed by measuring a binding property of the antibody of the present invention to human FCRL1 -expressing cells using ELISA, flow cytometry, a surface plasmon resonance method, or the like. Confirmation can also be performed in combination with a known immunological detection method [Monoclonal Antibodies-Principles and Practice, Third Edition, Academic Press (1996), Antibodies-A Laboratory Manual, Cold Spring Harbor Laboratory (1988), Monoclonal Antibody Laboratory Manual, Kodansha Scientific (1987)], and the like.

An antibody molecule is also referred to as an immunoglobulin (hereinafter, referred to as Ig), and the human antibody is classified into IgA1, IgA2, IgD, IgE, IgG1, IgG2, IgG3, IgG4, and IgM isotypes according to a difference in molecular structure. IgG1, IgG2, IgG3, and IgG4 having relatively high amino acid sequence similarity are also collectively referred to as IgG.

The antibody molecule is composed of polypeptides called a heavy chain (hereinafter, referred to as H chain) and a light chain (hereinafter, referred to as L chain). The H chain is composed of an H chain variable region (also referred to as VH) and an H chain constant region (also referred to as CH) from the N-terminal side, and the L chain is composed of an L chain variable region (also referred to as VL) and an L chain constant region (also referred to as CL) from the N-terminal side. As for CH, α, δ, ε, γ, and µ chains are known for each Ig isotype. CH is further composed of a CH1 domain, a hinge region, a CH2 domain, and a CH3 domain from the N-terminal side. The domain refers to a functional structural unit constituting each polypeptide of the antibody molecule. The CH2 domain and the CH3 domain are collectively referred to as an Fc region or a simply Fc. A Cλ chain and a Cκ chain are known as CL.

The CH1 domain, the hinge region, the CH2 domain, the CH3 domain, and the Fc region in the present invention can be specified by the number of the amino acid residue from the N-terminal by the EU index (also referred to as EU numbering) [Kabat et al., Sequences of Proteins of Immunological Interest, US Dept. Health and Human Services (1991)]. Specifically, the CH1 is identified as the amino acid sequences of EU index numbers 118 to 215, the hinge is identified as the amino acid sequences of EU index numbers 216 to 230, the CH2 is identified as the amino acid sequences of EU index numbers 231 to 340, and the CH3 is identified as the amino acid sequences of EU index numbers 341 to 447.

The monoclonal antibody in the present invention can include an antibody produced by a hybridoma and a recombinant antibody produced by a transformed cell transformed with an expression vector comprising an antibody gene.

The hybridoma refers to a cell that produces a monoclonal antibody having desired antigen specificity and is obtained by fusing a B cell obtained by immunizing a non-human animal with an antigen and a myeloma cell derived from a mouse or the like. Accordingly, the variable region constituting the antibody produced by the hybridoma consists of the amino acid sequence of a non-human animal antibody.

The antibody of the present invention also includes a recombinant antibody such as a recombinant mouse antibody, a recombinant rat antibody, a recombinant rabbit antibody, a human chimeric antibody (hereinafter, also simply referred to as a chimeric antibody), a humanized antibody (also referred to as a humanized complementarity determining region CDR-grafted antibody), and a human antibody, which are produced in a genetic engineering manner.

The chimeric antibody indicates an antibody consisting of a VH and a VL of an antibody from an animal other than human (non-human animal) and a CH and a CL of a human antibody. Any non-human animals can be used as long as hybridomas can be prepared, such as mice, rats, hamsters, and rabbits.

The human chimeric antibody can be produced by obtaining cDNAs each encoding a VH and a VL of the monoclonal antibody from monoclonal antibody-producing hybridomas derived from non-human animal cells, inserting the cDNAs into an animal cell expression vector comprising DNAs each encoding a CH and a CL of a human antibody, respectively, to construct a human chimeric antibody expression vector, and introducing the chimeric antibody expression vector into an animal cell to express the chimeric antibody.

The humanized antibody indicates an antibody obtained by grafting amino acid sequences of CDRs of a VH and a VL of a non-human animal antibody onto corresponding CDRs of a VH and a VL of a human antibody. A region other than CDRs of a VH and a VL is referred to as a framework region (hereinafter, referred to as FR).

The humanized antibody can be produced by constructing a cDNA encoding an amino acid sequence of a VH consisting of an amino acid sequence of CDR of a VH of a non-human animal antibody and an amino acid sequence of an FR of a VH of any human antibody, and a cDNA encoding an amino acid sequence of a VL consisting of an amino acid sequence of CDR of a VL of a non-human animal antibody and an amino acid sequence of an FR of a VH of any human antibody, inserting the cDNAs into an animal cell expression vector comprising DNAs each encoding a CH and a CL of a human antibody, respectively, to construct a humanized antibody expression vector, and introducing the humanized antibody expression vector into an animal cell to express the humanized antibody.

The human antibody originally refers to an antibody naturally occurring in the human body, and also includes an antibody obtained from a human antibody phage library prepared by recent advances in genetic engineering, cellular engineering, and developmental engineering technologies, and human antibody-producing transgenic animals.

The human antibody can be obtained by immunizing a mouse carrying a human immunoglobulin gene (Tomizuka K. et. al., Proc Natl Acad Sci USA. 97, 722-7, 2000) with a desired antigen. In addition, by using a phase display library obtained by amplifying an antibody gene from human-derived B cells, a human antibody having a desired binding activity is selected, and thus a human antibody can be obtained without immunization (Winter G. et. al., Annu Rev Immunol.12:433-55. 1994). Furthermore, by immortalizing human B cells using EB viruses, cells that produce a human antibody having a desired binding activity can be produced and a human antibody can be obtained (Rosen A. et. al., Nature 267, 52-54.1977).

For the antibody present in the human body, for example, lymphocytes that produce the antibody can be obtained by immortalizing lymphocytes isolated from human peripheral blood with EB viruses or the like and then cloning them, and the antibody can be purified from a culture of the lymphocytes.

The human antibody phage library is a phage library from which an antibody fragment such as Fab or scFv is expressed on a surface by inserting an antibody gene prepared from a human B cell into a phage gene. From the library, a phage expressing an antibody fragment having a desired antigen binding activity can be collected by using the binding activity to a substrate immobilized with an antigen as an indicator. The antibody fragment can also be further converted into a human antibody molecule consisting of two complete H chains and two complete L chains by a genetic engineering method.

The human antibody-producing transgenic animal refers to an animal obtained by incorporating a human antibody gene into a chromosome of a host animal. Specifically, the human antibody-producing transgenic animal can be produced by introducing a human antibody gene into a mouse ES cell, and grafting the ES cell into an early embryo of another mouse to produce an individual. As a method for producing a human antibody from the human antibody-producing transgenic animal, a human antibody-producing hybridoma is obtained by a hybridoma producing method performed in a mammal other than a normal human, and cultured to produce and accumulate a human antibody in a culture.

The amino acid sequences of the VH and the VL of the antibody of the present invention may be any of amino acid sequences of a VH and a VL of a human antibody, amino acid sequences of a VH and a VL of a non-human animal antibody, and amino acid sequences of a VH and a VL of a humanized antibody obtained by grafting CDRs of a non-human animal antibody onto a framework of any human antibody.

The amino acid sequence of the CL in the antibody of the present invention may be either an amino acid sequence of a human antibody or an amino acid sequence of a non-human animal antibody, and C_{κ} or C_{λ} of the amino acid sequences of the human antibody is preferred.

The CH of the antibody of the present invention may be any CH of molecular species belonging to immunoglobulins, and is preferably any of a subclass belonging to IgG class, γ1 (IgG1; for example, Accession Number: AAA02914.1), y2 (IgG2; for example, Accession Number: AAG00910.2), y3 (IgG3; for example, Accession Number: P01860.2), and y4 (IgG4; for example, Accession Number: P01861.1). The CH may be a CH in which one or more amino acids constituting the CH are deleted, substituted, or added. The number of amino acids to be deleted, substituted, or added is not particularly limited, and is preferably 1 to several tens of amino acids, for example 1 to 20 amino acids, and more preferably 1 to several amino acids, for example 1 to 5 amino acids. Examples of the CH in which one or more amino acids constituting the CH are deleted, substituted, or added include an IgG1 CH variant obtained by substituting, with cysteine, serine in a human IgG1 CH at position 239 according to EU numbering. More specific examples thereof include an IgG1 CH variant comprising the amino acid sequence (SEQ ID NO: 80) in which serine in human IgG1 CH comprising the amino acid sequence represented by SEQ ID NO: 79 at position 239 according to EU numbering is substituted with cysteine.

The antibody of the present invention also includes an Fc fusion protein obtained by binding Fc and an antibody fragment to each other, an Fc fusion protein obtained by binding Fc and a naturally occurring ligand or a receptor to each other (also referred to as immunoadhesin), an Fc fusion protein obtained by fusing a plurality of Fc regions, and the like. In order to stabilize the antibody and control the blood half-life, an Fc region with a modified amino acid residue can also be used for the antibody of the present invention.

The antibody or the antibody fragment thereof of the present invention also includes an antibody comprising any post-translationally modified amino acid residue. Examples of the post-translational modification include deletion of a lysine residue of an H chain at the C-terminus (lysine clipping) and substitution of a glutamine residue of the polypeptide with pyroglutamine (pyroGlu) at the N-terminus [Beck et al, Analytical Chemistry, 85, 715-736 (2013)].

In the present invention, the antibody fragment is an antibody fragment that has an antigen binding activity and binds to the extracellular region of the human FCRL 1. Examples of the antibody fragment in the present invention include Fab, Fab', F(ab')₂, scFv, diabody, dsFv, and a peptide comprising CDR. Fab is an antibody fragment obtained by treating an IgG antibody with a proteolytic enzyme papain (obtained by cleaving at an amino acid residue at position 224 in an H chain) and having a molecular weight of about 50000 and an antigen binding activity, in which about half of the H chain on an N-terminal side and an entire L chain bind to each other by a disulfide bond (S-S bond). The antibody fragment of the present invention is preferably an antibody fragment that binds to an extracellular region of FCRL 1 and induces internalization of FCRL 1.

The F(ab')₂ is an antibody fragment obtained by treating an IgG antibody with a proteolytic enzyme pepsin (obtained by cleaving at an amino acid residue at position 234 in an H chain) and having a molecular weight of about 100000 and an antigen binding activity, which is slightly larger than that of Fab bound through an S-S bond in a hinge region. The Fab' is an antibody fragment obtained by cleaving the S-S bond in the hinge region of the F(ab')₂ and having a molecular weight of about 50000 and an antigen binding activity.

The scFv is a VH-P-VL or VL-P-VH polypeptide obtained by linking one VH and one VL by a suitable peptide linker (P) such as a linker peptide in which any number of linkers (G4S) consisting of 4 Gly and 1 Ser residues are connected, and is an antibody fragment having an antigen binding activity.

The Diabody is an antibody fragment obtained by forming a dimer with scFvs having the same or different antigen binding specificity, and is an antibody fragment having an divalent antigen binding activity to the same antigen or a specific antigen binding activity to different antigens.

The dsFv refers to a polypeptide in which one amino acid residue each in a VH and a VL is substituted with a cysteine residue, which are linked via an S-S bond between the cysteine residues.

The peptide comprising CDR includes at least one region of a CDR of a VH or a VL. In the peptide comprising a plurality of CDRs, the CDRs can be linked directly or via a suitable peptide linker. The peptide comprising CDR can be produced by constructing DNAs each encoding CDRs of a VH and a VL of the modified antibody of the present invention, inserting the DNAs into a prokaryotic expression vector or an eukaryotic expression vector, and introducing the expression vector into prokaryotes or eukaryotes to express the peptide comprising CDR. The peptide comprising CDR can also be produced by a chemical synthesis method such as an Fmoc method or a tBoc method.

One aspect of the antibody of the present invention includes any one selected from the following (a) to (g):
(a) an antibody in which complementarity determining regions (hereinafter, abbreviated as CDR) 1 to 3 of a heavy chain variable region (hereinafter, abbreviated as VH) comprise the amino acid sequences represented by SEQ ID NOs: 20 to 22, respectively, and CDRs 1 to 3 of a light chain variable region (hereinafter, abbreviated as VL) comprise the amino acid sequences represented by SEQ ID NOs: 24 to 26, respectively,
(b) an antibody in which CDRs 1 to 3 of a VH comprise the amino acid sequences represented by SEQ ID NOs: 28 to 30, respectively, and CDRs 1 to 3 of a VL comprise the amino acid sequences represented by SEQ ID NOs: 32 to 34, respectively,
(c) an antibody in which CDRs 1 to 3 of a VH comprise the amino acid sequences represented by SEQ ID NOs: 36 to 38, respectively, and CDRs 1 to 3 of a VL comprise the amino acid sequences represented by SEQ ID NOs: 40 to 42, respectively,
(d) an antibody in which CDRs 1 to 3 of a VH comprise the amino acid sequences represented by SEQ ID NOs: 44 to 46, respectively, and CDRs 1 to 3 of a VL comprise the amino acid sequences represented by SEQ ID NOs: 48 to 50, respectively,
(e) an antibody in which CDRs 1 to 3 of a VH comprise the amino acid sequences represented by SEQ ID NOs: 52 to 54, respectively, and CDRs 1 to 3 of a VL comprise the amino acid sequences represented by SEQ ID NOs: 56 to 58, respectively, and
(f) an antibody in which CDRs 1 to 3 of a VH comprise the amino acid sequences represented by SEQ ID NOs: 60 to 62, respectively, and CDRs 1 to 3 of a VL comprise the amino acid sequences represented by SEQ ID NOs: 64 to 66, respectively, and
(g) an antibody in which CDRs 1 to 3 of a VH comprise the amino acid sequences represented by SEQ ID NOs: 36 to 38, respectively, and CDRs 1 to 3 of a VL comprise the amino acid sequences represented by SEQ ID NOs: 40, 71, and 42, respectively.

One aspect of the antibody of the present invention includes any one selected from the following (1a) to (1f):
(1a) an antibody in which a VH comprises the amino acid sequence represented by SEQ ID NO: 19 and a VL comprises the amino acid sequence represented by SEQ ID NO: 23,
(1b) an antibody in which a VH comprises the amino acid sequence represented by SEQ ID NO: 27 and a VL comprises the amino acid sequence represented by SEQ ID NO: 31,
(1c) an antibody in which a VH comprises the amino acid sequence represented by SEQ ID NO: 35 and a VL comprises the amino acid sequence represented by SEQ ID NO: 39,
(1d) an antibody in which a VH comprises the amino acid sequence represented by SEQ ID NO: 43 and a VL comprises the amino acid sequence represented by SEQ ID NO: 47,
(1e) an antibody in which a VH comprises the amino acid sequence represented by SEQ ID NO: 51 and a VL comprises the amino acid sequence represented by SEQ ID NO: 55, and
(1f) an antibody in which a VH comprises the amino acid sequence represented by SEQ ID NO: 59 and a VL comprises the amino acid sequence represented by SEQ ID NO: 63.

As one aspect of the antibody of the present invention, examples thereof include anti-human FCRL1 mouse monoclonal antibodies DK610, DK681, DK1142, DK1141, DK1166, and DK1164, which will be described later in Examples. As one aspect of the antibody of the present invention, examples thereof include an antibody comprising a variable region of any one of DK610, DK681, DK1142, DK1141, DK1166, and DK1164. As one aspect of the antibody of the present invention, examples thereof include an antibody comprising amino acid sequences of CDRs 1 to 3 of a VH and CDRs 1 to 3 of a VL of any one antibody of DK610, DK681, DK1142, DK1141, DK1166, and DK1164.

As one aspect of the antibody of the present invention, examples thereof include any one selected from the following (2b-1) to (2b-4), (2c-1), (2c-2), and (2g-1):
(2b-1) an antibody in which a VH comprises the amino acid sequence represented by SEQ ID NO: 72 and a VL comprises the amino acid sequence represented by SEQ ID NO: 68,
(2b-2) an antibody in which a VH comprises the amino acid sequence represented by SEQ ID NO: 73 and a VL comprises the amino acid sequence represented by SEQ ID NO: 74,
(2b-3) an antibody in which a VH comprises the amino acid sequence represented by SEQ ID NO: 72 and a VL comprises the amino acid sequence represented by SEQ ID NO: 74,
(2b-4) an antibody in which a VH comprises the amino acid sequence represented by SEQ ID NO: 73 and a VL comprises the amino acid sequence represented by SEQ ID NO: 68,
(2c-1) an antibody in which a VH comprises the amino acid sequence represented by SEQ ID NO: 75 and a VL comprises the amino acid sequence represented by SEQ ID NO: 76,
(2c-2) an antibody in which a VH comprises the amino acid sequence represented by SEQ ID NO: 77 and a VL comprises the amino acid sequence represented by SEQ ID NO: 76, and
(2g-1) an antibody in which a VH comprises the amino acid sequence represented by SEQ ID NO: 77 and a VL comprises the amino acid sequence represented by SEQ ID NO: 78.

As one aspect of the antibody of the present invention, examples thereof include a humanized antibody obtained by grafting amino acid sequences of CDRs 1 to 3 of a VH and CDRs 1 to 3 of a VL of a DK681 antibody or a DK1142 antibody into an FR of a human antibody. Examples of such an antibody include DK681 F11, DK681 F12, DK681 F13, DK681 F14, DK1142 F21, and DK1142 F22, which will be described later in Examples. Examples of the humanized antibody obtained by grafting amino acid sequences of CDRs 1 to 3 of a VH and CDR 1 and 3 of a VL of a DK1142 antibody and an amino acid sequence obtained by modifying CDR 2 of a VL of the DK1142 antibody into an FR of a human antibody include DK1142 F24, which will be described later in Examples.

Examples of the antibody of the present invention include an antibody that selectively binds to FCRL 1 expressed on the cell surface and causes internalization of FCRL 1. Examples of the antibody of the present invention include an antibody exhibiting a strong drug effect when a drug is bound to the antibody to obtain an ADC.

The fact that the antibody of the present invention induces internalization of FCRL 1 can be confirmed by, for example, adding a reagent that emits fluorescence in a low pH environment such as an intracellular lysosome to cells after binding the reagent to the antibody and measuring a fluorescence intensity.

The antibody of the present invention also includes an antibody into which a chemical structure capable of reacting with a drug or a linker to form a bond is introduced. Examples thereof include an antibody obtained by adding, inserting, or substituting a natural or non-natural amino acid residue having a functional group such as an α, β-unsaturated carbonyl group, an α, β-unsaturated sulfinyl group, an α, β-unsaturated sulfonyl group, a thiol group, a hydroxyl group, an amino group, an amide group, a formyl group, a carboxyl group, an azide group, an alkynyl group, an alkenyl group, a haloalkyl group, or a carbonyl group into the N-terminal, the C-terminal, or in the middle of the amino acid sequence of a heavy chain or a light chain of an antibody, and an antibody obtained by introducing a sugar chain having a functional group such as an α, β-unsaturated carbonyl group, an α, β-unsaturated sulfinyl group, an α, β-unsaturated sulfonyl group, a thiol group, a hydroxyl group, an amino group, an amide group, a formyl group, a carboxyl group, an azide group, an alkynyl group, an alkenyl group, a haloalkyl group, or a carbonyl group into a sugar chain of the antibody.

Examples of the antibody include an antibody obtained by substituting an amino acid residue of the antibody at a specific position with cysteine. Examples of the amino acid residue of a heavy chain suitable for substitution with cysteine in the IgG antibody include at least one of serine at position 239 according to EU numbering (Dimasi, N. et. al., Molecular Pharmaceutics. 14, 1501-1516, 2017), serine at position 442 (Stimmel, JB. et. al., The Journal of Biological Chemistry. 275, 30445-50, 2000), lysine at position 290 (Graziani, EI. et. al., Molecular Cancer Therapeutics. 19, 2068-2078, 2020), threonine at position 114, alanine at position 140, leucine at position 174, leucine at position 179, threonine at position 187, threonine at position 209, valine at position 262, glycine at position 371, tyrosine at position 373, glutamic acid at position 382, serine at position 424, asparagine at position 434, and glutamine at position 438 (WO2016/040856). Examples of the amino acid residue of a κ light chain suitable for substitution with cysteine include at least one of lysine at position 183 according to EU numbering (Graziani, EI. et. al., Molecular Cancer Therapeutics. 19, 2068-2078, 2020), glutamine at position 124 (Shinmi, D. et. al., Bioconjugate Chemistry. 27, 1324-31, 2016), leucine or isoleucine at position 106, arginine at position 108, arginine at position 142, and lysine at position 149 (WO2016/040856).

Examples thereof include an antibody introduced with paraacetylphenylalanine (Skidmore, L. et. al., Molecular Cancer Therapeutics 19(9), 1833-1843, 2020), an antibody in which a thiol group of a cysteine residue is enzymatically converted to a formyl group (U.S. Patent Application Publication No. 2012/0183566), and an antibody in which cysteine is inserted between serine at position 239 and valine at position 240 in a heavy chain constant region according to EU numbering (U.S. Patent No. 10744204).

The ADC containing the antibody of the present invention contains a molecule in which the antibody and a drug are bound to each other directly or via a linker chemically or through genetic engineering. Such an antibody portion in the ADC molecule is also included in the antibody of the present invention.

The drug contained in the ADC of the present invention (also referred to as a payload in the present description) may be any molecule as long as it has a physiological activity, and examples thereof include a radioactive isotope, a low-molecular drug, a high molecular drug, a protein, an antibody drug, and a nucleic acid drug.

The ADC can be produced by linking a drug or a linker to the N-terminus or C-terminus of the H chain or L chain of the antibody or the antibody fragment thereof that binds to human FCRL1 of the invention, or to an appropriate functional group or a side chain or a sugar chain in the antibody molecule by a chemical method [Introduction of Antibody Engineering, Chijin Shoin (1994)].

The antibody or the antibody fragment thereof of the present invention can be linked to a drug or a linker by a known method (for example, a method described in S. J. Walsh et al. Chem. Soc. Rev. 2021, 50, 1305-1353; Tumey, L. Nathan (2020). Antibody-Drug Conjugates -Methods and Protocols: New York, Springer; Laurent Ducry (2013). Antibody-Drug Conjugate: New York, Springer, and the like). Examples thereof include a method in which a functional group introduced into an antibody molecule (including a sugar chain binding to an antibody), such as an α, β-unsaturated carbonyl group, an α, β-unsaturated sulfinyl group, an α, β-unsaturated sulfonyl group, a thiol group, a hydroxyl group, an amino group, an amide group, a formyl group, a carboxyl group, an azide group, an alkynyl group, an alkenyl group, a haloalkyl group, or a carbonyl group, is reacted with a functional group contained in a drug or a linker under appropriate conditions. The combination of the functional group contained in the antibody and the functional group contained in the drug or linker can be more appropriately selected based on known information. For example, a bond can be formed by a nucleophilic reaction between a nucleophilic functional group in the antibody molecule, such as an alkol group, and a Michael acceptor such as an α, β-unsaturated carboxylic acid contained in the drug or linker. For example, a bond can be formed by cyclizing an azide group in the antibody molecule and an alkynyl group contained in the drug or linker in the presence or absence of a catalyst.

The ADC can be produced by a genetic engineering method in which a DNA encoding the monoclonal antibody or the antibody fragment thereof that binds to human FCRL 1 of the present invention and a DNA encoding a protein or an antibody drug to be bound are connected and inserted into an expression vector, and the expression vector is introduced into a suitable host cell to express to the ADC.

Examples of the radioactive isotope include 111In, 131I, 125I, 90Y, 64Cu, 99Tc, 77Lu, and 211At. The radioactive isotope can directly bind to the antibody by a chloramine T method or the like. A substance for chelating the radioactive isotope may bind to the antibody. Examples of the chelating reagent include 1-isothiocyanatebenzyl-3-methyldiethylenetriaminepentaacetic acid (MX-DTPA).

Examples of the low-molecular drug include an alkylating agent, nitrosourea, an antimetabolite, an antibiotic, a plant alkaloid, a topoisomerase inhibitor, a hormone therapy agent, a hormone antagonist, an aromatase inhibitor, a P-glycoprotein inhibitor, a platinum complex derivative, an anticancer agent such as an M-phase inhibitor or a kinase inhibitor [Clinical Oncology, Cancer and Chemotherapy (1996)], a steroidal drug such as hydrocortisone or prednisone, a non-steroidal drug such as aspirin or indomethacin, an immunomodulator such as gold thiomalate or penicillamine, an immunosuppressant such as cyclophosphamide or azathioprine, and an anti-inflammatory agent such as an antihistamine such as chlorpheniramine maleate or clemacitin [Inflammation and anti-inflammatory therapy, Ishiyaku Pub, Inc. (192)].

Examples of the anticancer agent include amifostine (ethiol), cisplatin, dacarbazine (DTIC), dactinomycin, mechlorethamine (nitrogen mustard), streptozocin, cyclophosphamide, ifosfamide, carmustine (BCNU), lomustine (CCNU), doxorubicin (adriamycin), epirubicin, gemcitabine (Gemzar), daunorubicin, procarbazine, mitomycin, cytarabine, etoposide, methotrexate, 5-fluorouracil, fluorouracil, vinblastine, vincristine, bleomycin, daunomycin, pepromycin, estramustine, paclitaxel (Taxol), docetaxel (Taxotea), Aldesleukin, asparaginase, busulfan, carboplatin, oxaliplatin, Nedaplatin, cladribine, camptothecin, 10-hydroxy-7-ethyl-camptothecin (SN38), Floxuridine, fludarabine, hydroxyurea, idarubicin, mesna, irinotecan (CPT-11), topotecan, mitoxantrone, topotecan, leuprolide, megestrol, melphalan, mercaptopurine, hydroxycarbamide, plicamycin, mitotane, pegasparagase, pentostatin, pipobroman, Tamoxifen, Goserelin, Leuprorenin, Flutamide, Teniposide, Testolactone, thioguanine, thiotepa, uracil mustard, vinorelbine, chlorambucil, hydrocortisone, prednisolone, methylprednisolone, vindesine, nimustine, semustine, capecitabine, tomdex, azacytidine, oxaloplatin, a pyrrolobenzodiazepine (PBD) derivative, auristatins (monomethyl auristatin E, monomethyl auristatin F, etc.), amanitin, camptothecin derivatives (deruxtecan, exatecan, SN-38, etc.), Gefitinib (Iressa), Imatinib (STI571), Erlotinib, an FMS-like tyrosine kinase 3 (Flt3) inhibitor, a vascular endothelial growth factor receptor (VEGFR) inhibitor, a fibroblast growth factor receptor (FGFR) inhibitor, an epidermal growth factor receptor (EGFR) inhibitor such as Iressa or Tarceva, radicicol, 17-allylamino-17-demethoxygeldanamycin, rapamycin, amsacrine, all-trans retinoic acid, thalidomide, lenalidomide, anastrozole, fadrozole, letrozole, exemestane, gold thiomalate, D-penicillamine, bucillamine, azathioprine, mizoribine, cyclosporine, hydrocortisone, Bexarotene (Targretin), Tamoxifen, Dexamethasone, progestins, estrogens, anastrozole (arimidex), leupurin, aspirin, indomethacin, celecoxib, penicillamine, gold thiomalate, chlorpheniramine maleate, chloropheniramine, clemacitin, tretinoin, bexarotene, arsenic, bortezomib, allopurinol, ibritumomab tiuxetan, targretin, ozogamine, clarithromasin, leucovorin, ketoconazole, aminoglutethimide, suramin, or maytansinoid, dolastatin 10, actinomycin, anthracycline, duocarmycin, duocarmycin dimer (CPI-dimer, etc.), eribulin or a derivative thereof.

Examples of the high-molecular drug include polyethylene glycol (hereinafter, referred to as PEG), albumin, dextran, polyoxyethylene, a styrene maleic acid copolymer, polyvinylpyrrolidone, a pyran copolymer, or hydroxypropyl methacrylamide. By linking the high molecular weight compound to the antibody or the antibody fragment thereof, effects such as (1) improvement of stability with respect to various chemical, physical, or biological factors, (2) significant extension of blood half-life, and (3) loss of immunogenicity or prevention of antibody production are expected [Bioconjugate pharmaceuticals, Hirokawa Shoten (1993)].

Examples of the method of linking a PEG and the antibody include a method for reacting with a PEGylation modification reagent [Bioconjugate pharmaceuticals, Hirokawa Shoten (1993)]. Examples of the PEGylation modification reagent include a modifier for an ε-amino group of lysine (JP61-178926A), a modifier for a carboxy group of aspartic acid and glutamic acid (JP56-23587A), or a modifier for a guanidino group of arginine (JP2-117920A).

The immunostimulant may be a natural product known as an immunoadjuvant, and specific examples thereof include drugs that enhance immunity, such as β(1→3) glucan (for example, lentinan or schizophyllan), or α-galactosylceramide (KRN7000).

Examples of the protein include a cytokine or a growth factor that activates immunocompetent cells such as NK cells, macrophages, or neutrophils, or a toxin protein.

Examples of the cytokine or growth factor include interferon (hereinafter referred to as IFN)-α, IFN-β, IFN-y, interleukin (hereinafter referred to as IL)-2, IL-12, IL-15, IL-18, IL-21, and IL-23, a granulocyte-colony stimulating factor (G-CSF), a granulocyte/macrophage-colony stimulating factor (GM-CSF), or a macrophage-colony stimulating factor (M-CSF). Examples of the toxin protein include ricin, or diphtheria toxin, and the toxin protein also includes a protein toxin obtained by introducing a mutation into a protein to adjust toxicity.

Examples of the antibody drug include an antibody against an antigen that induces apoptosis upon antibody binding, an antigen that is associated with pathogenesis of tumors, an antigen that regulates an immune function, and an antigen that is associated with angiogenesis at a lesion site.

Examples of the antigen that induces apoptosis upon antibody binding include a cluster of differentiation (hereinafter referred to as CD) 19, CD20, CD21, CD22, CD23, CD24, CD37, CD53, CD72, CD73, CD74, CDw75, CDw76, CD77, CDw78, CD79a, CD79b, CD80 (B7.1 ), CD81, CD82, CD83, CDw84, CD85, CD86 (B7.2), human leukocyte antigen (HLA)-Class II, or Epidermal Growth Factor Receptor (EGFR).

Examples of the antigen that is associated with pathogenesis of tumors or the antigen of an antibody that regulates an immune function include CD4, CD40, a CD40 ligand, a B7 family molecule (for example, CD80, CD86, CD274, B7-DC, B7-H2, B7-H3, or B7-H4), a B7 family molecule ligand (for example, CD28, CTLA-4, ICOS, PD-1, or BTLA), OX-40, an OX-40 ligand, CD 137, a tumor necrosis factor (TNF) receptor family molecule (for example, DR4, DR5, TNFR1, or TNFR2), a TNF-related apoptosis-inducing ligand receptor (TRAIL) family molecule, a TRAIL family molecule receptor family (for example, TRAIL-R1, TRAIL-R2, TRAIL-R3, or TRAIL-R4), a receptor activator of nuclear factor kappa B ligand (RANK), a RANK ligand, CD25, a folate receptor, cytokines [for example, IL-1α, IL-1β, IL-4, IL-5, IL-6, IL-10, IL-13, transforming growth factor (TGF)β or TNFα] or receptors for these cytokines, or chemokines (for example, SLC, ELC, I-309, TARC, MDC, or CTACK), or receptors for these chemokines.

Examples of the antigen of an antibody that inhibits angiogenicity of a lesion site include a vascular endothelial growth factor (VEGF), an angiopoietin, a fibroblast growth factor (FGF), EGF, a hepatocyte growth f factor (HGF), a platelet-derived growth factor (PDGF), an insulin-like growth factor (IGF), erythropoietin (EPO), TGFβ, IL-8, ephrin, SDF-1, or receptors thereof.

For a fusion antibody with a protein or an antibody drug, the fusion antibody can be produced by connecting a cDNA encoding an antibody contained in the protein or antibody drug to a cDNA encoding the monoclonal antibody or the antibody fragment thereof to construct a DNA encoding the fusion antibody, inserting the DNA into an expression vector for prokaryotes or eukaryotes, and introducing the expression vector into prokaryotes or eukaryotes to express the fusion antibody.

Examples of the nucleic acid drug include a drug medicine such as small interference ribonucleic acid (siRNA) or microRNA that act on living organisms by controlling gene functions. For example, a conjugate with a nucleic acid drug that prevents a master transcription factor RORyt of Th17 cells can be considered.

The linker contained in the ADC of the present invention may have any structure as long as it has a function of linking an antibody and a drug. For example, the linker may have a structure having a special function such as cutting in the vicinity or inside of a target cell or tissue, or a branched structure capable of linking a plurality of drugs. For the ADC of the present invention, for example, a known linker (for example, linkers described in S. J. Walsh et al. Chem. Soc. Rev. 2021, 50, 1305-1353; Tumey, L. Nathan (2020). Antibody-Drug Conjugates-Methods and Protocols: New York, Springer; and Laurent Ducry (2013). Antibody-Drug Conjugate: New York, Springer) can be used. Specific examples thereof include a linker composed of any one selected from the group consisting of a peptide, an oligosaccharide, -(CH₂)-, an oxygen atom, a sulfur atom, -NH-, -(CH₂CH₂O)-, -CO-, -PO-, an amino acid, para-amino benzyl (PAB), a cyclic alkyl having 3 to 10 carbon atoms, and a structure represented by the following formula, and a linker containing a structure obtained by linking two or more units selected from the above-described groups

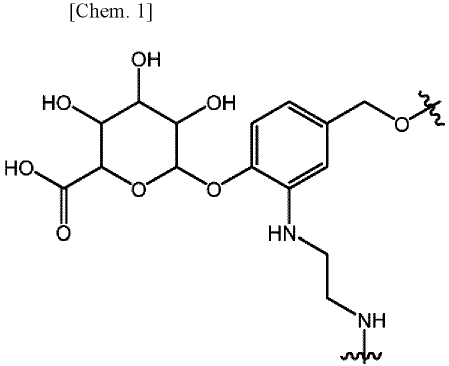

Examples of the amino acid constituting the linker include valine (Val), citrulline (Cit), phenylalanine (Phe), lysine (Lys), D-valine (D-Val), leucine (Leu), glycine (Gly), alanine (Ala), and asparagine (Asn).

As one aspect of the linker, examples thereof include a linker containing any one selected from the group consisting of a peptide, an oligosaccharide, -(CH₂)ₙ-, -(CH₂CH₂O)ₙ-, -CO-, Val-Cit-PAB, Val-Ala-PAB, Val-Lys(Ac)-PAB, Phe-Lys-PAB, Phe-Lys(Ac)-PAB, Ala-PAB, PAB, D-Val-Leu-Lys, Gly-Gly-Arg, Ala-Ala-Asn-PAB, Gly-Gly-Phe-Gly-PAB, -Gly-Gly-Phe-Gly-CH₂-O-CH₂-CO-, and a structure represented by the following formula, and a linker containing a structure obtained by linking two or more units selected from the above-described group.

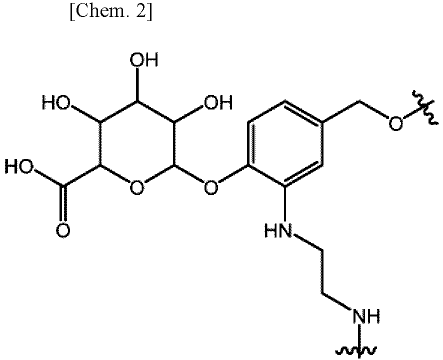

Here, n represents an integer of 1 to 1000, preferably represents an integer of 1 to 100, more preferably represents an integer of 1 to 50, further preferably represents an integer of 1 to 20, and most preferably represents an integer of 1 to 15. Ac represents an acetyl group. Lys(Ac) represents that an amino group of lysine in a side chain is acetylated.

As one aspect of the linker, examples thereof include a linker containing any one of -(CH₂)ₘ-CO-NH-(CH₂CH₂O)ₙ-Val-Cit-PAB, -(CH₂)ₘ-CO-NH-(CH₂CH₂O)ₙ-Val-Ala-PAB, -(CH₂)ₘ-CO-NH-(CH₂CH₂O)ₙ-Val-Lys(Ac)-PAB, -(CH₂)ₘ-CO-NH-(CH₂CH₂O)ₙ-Phe-Lys-PAB, -(CH₂)ₘ-CO-NH-(CH₂CH₂O)ₙ-Phe-Lys(Ac)-PAB, - (CH₂)ₘ-CO-NH-(CH₂CH₂O)ₙ-Ala-PAB, -(CH₂)ₘ-CO-NH-(CH₂CH₂O)ₙ-D-Val-Leu-Lys, -(CH₂)ₘ-CO-NH-(CH₂CH₂O)ₙ-Gly-Gly-Arg, -(CH₂)ₘ-CO-NH-(CH₂CH₂O)ₙ-Ala-Ala-Asn-PAB, -(CH₂)ₘ-NH-CO-cBu-CO-Cit-PAB, and -(CH₂)ₘ-Gly-Gly-Phe-Gly-CH₂-O-CH₂-CO-. Here, m represents an integer of 1 to 10, preferably represents 1. n represents an integer of 1 to 1000, preferably represents an integer of 1 to 100, more preferably represents an integer of 1 to 50, further preferably represents an integer of 1 to 20, and most preferably represents an integer of 1 to 15. Ac represents an acetyl group. Lys(Ac) represents that an amino group of lysine in a side chain is acetylated.

The linker before linking to the antibody preferably has a functional group capable of linking to the antibody and the drug. Examples of such a functional group include an α, β-unsaturated carbonyl group, an α, β-unsaturated sulfinyl group, an α, β-unsaturated sulfonyl group, a thiol group, an amino group, a hydroxy amino group, a hydrazide group, a hydrazyl group, an amide group, a formyl group, a carboxyl group, an azide group, an alkynyl group, an alkenyl group, and a haloalkyl group. Examples of an atom adjacent to a carbonyl carbon atom of an α, β-unsaturated carbonyl group, an amido group, and a carboxyl group, and a molecule adjacent to a sulfur atom of an α, β-unsaturated carbonyl group and an α, β-unsaturated sulfinyl group include carbon, oxygen, nitrogen, and a sulfur atom. Examples of the above-described α, β-unsaturated carbonyl group include a maleimide group. Examples of the above-described alkenyl group include a vinylpyridyl group. Examples of the above-described alkynyl group include a BCN group (Bicyclo "6.1.0" non-4-yne) and a dibenzocyclooctyne (DBCO) group.

A linker drug portion other than the antibody in the ADC of the present invention is also referred to as a linker payload. Examples of the linker payload of the present invention include a PBD dimer payload linker such as SG3249 represented by the following formula (Med. Chem. Lett. 2016, 7, 983-987).

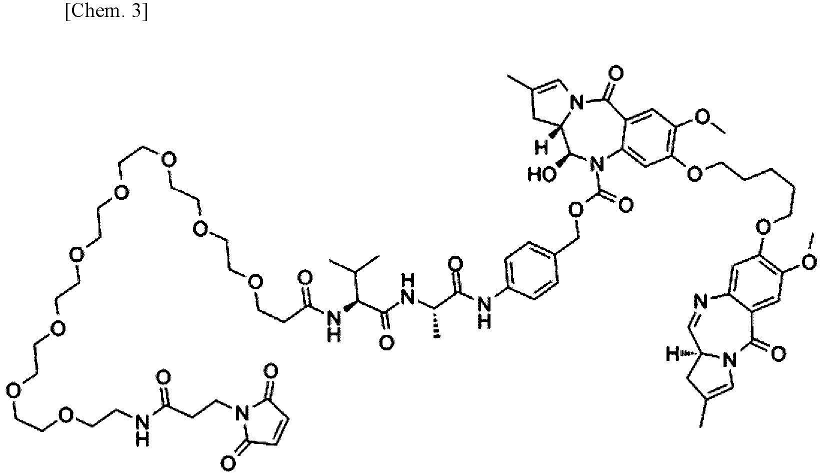

When a derivative of the antibody of the present invention is used for detection and measurement of human FCRL 1 and diagnosis of a disease associated with human FCRL 1, examples of the drug linking to the antibody include a labeling material used in ordinary immunological detection or measurement methods. Examples of the labeling material include an enzyme such as alkaline phosphatase, peroxidase, or luciferase, a luminescent substance such as an acridinium ester or a lophine, or a fluorescent substance such as fluorescein isothiocyanate (FITC) or tetramethylrhodamine isothiocyanate (RITC).

The present invention also includes a composition containing, as an active ingredient, the monoclonal antibody or the antibody fragment thereof that binds to human FCRL 1.

The present invention also relates to a therapeutic agent for a disease associated with human FCRL 1 that contains, as an active ingredient, the monoclonal antibody or the antibody fragment thereof that binds to human FCRL 1. The present invention also relates to a therapeutic method for a disease associated with human FCRL 1 that includes administering the monoclonal antibody or the antibody fragment thereof that binds to human FCRL 1.

The disease associated with human FCRL1 may be any disease that is associated with human FCRL1 or a ligand for human FCRL 1, and examples thereof include cancer, an autoimmune disease, and an inflammatory disease. Examples of the cancer disease include diffuse large B-cell lymphoma, follicular lymphoma, B-cell lymphoma, Hodgkin lymphoma, chronic lymphocytic leukemia, hairy cell leukemia, mantle cell lymphoma, follicular marginal zone lymphoma, and small lymphocytic lymphoma. Examples of the autoimmune disease and the inflammatory disease include rheumatoid arthritis, multiple sclerosis, chronic obstructive pulmonary disease, systemic lupus erythematosus, lupus nephritis, asthma, atopic inflammatory bowel disease, a Crohn's disease, or a Behcet's disease.

The therapeutic agent containing the antibody or the antibody fragment thereof of the present invention may contain only the antibody or the antibody fragment thereof as an active ingredient, and it is preferable that the therapeutic agent is mixed together with one or more pharmacologically acceptable carriers and provided as a pharmaceutical formulation prepared by any method known in the pharmaceutical art.

It is preferred to use a most effective route of administration for treatment. Examples thereof include oral administration, and parenteral administration such as buccal, tracheobronchial, intrarectal, subcutaneous, intramuscular, or intravenous administration. Preferred examples thereof includes intravenous administration. Examples of a dosage form include sprays, capsules, tablets, powders, granules, syrups, emulsions, suppositories, injections, ointments, and tapes.

A dosage or the number of doses varies depending on a desired therapeutic effect, an administration method, a treatment period, an age, a body weight, or the like, and is generally 10 µg/kg to 10 mg/kg per day for adults.

The present invention relates to a reagent for detecting or measuring FCRL 1 that contains the monoclonal antibody or the antibody fragment thereof that binds to human FCRL 1. The present invention relates to a method for detecting or measuring human FCRL1 using the monoclonal antibody or the antibody fragment thereof that binds to human FCRL1. As a method for detecting or measuring human FCRL1 in the present invention, any known method can be used. Examples thereof include an immunological detection or measurement method.

The immunological detection or measurement method is a method for detecting or measuring an antibody amount or antigen amount using a labeled antigen or antibody. Examples of the immunological detection or measurement method include a radiolabeled immune antibody method (RIA), enzyme immunoassay (EIA or ELISA), fluorescence immunoassay (FIA), luminescent immunoassay, Western blotting, or a physicochemical method.

The present invention relates to a diagnostic agent for a disease associated with FCRL 1 that contains the monoclonal antibody or the antibody fragment thereof that binds to human FCRL1, and a diagnostic method for a disease associated with FCRL1 that includes detecting or measuring FCRL1 using the monoclonal antibody or the antibody fragment thereof that binds to human FCRL1. The disease associated with human FCRL1 can be diagnosed by detecting or measuring cells expressing human FCRL1 according to the above method using the monoclonal antibody or the antibody fragment thereof of the present invention.

In the present invention, a biological sample to be detected or measured for human FCRL1 is not particularly limited as long as it may contain human FCRL 1 or cells expressing human FCRL 1, such as a tissue, cells, blood, plasma, serum, a pancreatic fluid, urine, feces, a tissue fluid, or a culture medium.

A diagnostic agent containing the monoclonal antibody or the antibody fragment thereof of the present invention may contain a reagent for performing an antigen-antibody reaction and a detection reagent for the reaction, depending on an intended diagnostic method. Examples of the reagent for performing an antigen-antibody reaction include a buffer and a salt. Examples of the detection reagent include a labeled secondary antibody that recognizes the monoclonal antibody or the antibody fragment thereof, or a reagent used in ordinary immunological detection or measurement methods, such as a substrate compatible with labels.

The present invention relates to use of an anti-human FCRL1 monoclonal antibody or an antibody fragment thereof for producing a therapeutic agent or a diagnostic agent for a disease associated with FCRL 1.

Hereinafter, the method for producing the antibody, the therapeutic method for a disease, and the diagnostic method for a disease of the present invention will be specifically described.

### 1. Method for Producing Antibody

### (1) Preparation of Antigen

Human FCRL1 as an antigen or human FCRL 1-expressing cells can be obtained by introducing an expression vector comprising a cDNA encoding full-length human FCRL 1 or a partial length thereof into Escherichia coli, yeast, insect cells, animal cells, or the like. The human FCRL 1 can also be obtained by purifying human FCRL 1 from various human cell lines, human cells, human tissues, and the like that express large amounts of human FCRL 1. The human cell lines, human cells, human tissues, and the like may be used as antigens as they are. Further, a synthetic peptide having a partial sequence of the human FCRL1 can be prepared by a chemical synthesis method such as an Fmoc method or a tBoc method and used as an antigen. A known tag such as FLAG or His may be added to the human FCRL 1 or a synthetic peptide having a partial sequence of the human FCRL 1 at the C-terminal or N-terminal.

The human FCRL1 used in the present invention can be produced by expressing a DNA encoding the human FCRL1 in a host cell using a method described in Molecular Cloning, A Laboratory Manual, Second Edition, Cold Spring Harbor Laboratory Press (1989) and Current Protocols In Molecular Biology, John Wiley & Sons (1987-1997), and the like, for example, by the following method.

First, a recombinant vector is prepared by inserting a full-length cDNA comprising a portion encoding the human FCRL1 downstream of a promoter of an appropriate expression vector Instead of the full-length cDNA described above, a DNA fragment of an appropriate length prepared based on the full-length cDNA and comprising a portion encoding a polypeptide may be used. Next, by introducing the obtained recombinant vector into a host cell compatible with the expression vector, a transformant that produces a polypeptide can be obtained.

Any expression vectors can be used as long as it is capable of autonomous replication in a host cell to be used or integration into a chromosome, and contains an appropriate promoter at a position where a DNA encoding a polypeptide can be transcribed. Any host cells can be used as long as it can express a target gene, such as microorganisms belonging to the genus Escherichia such as Escherichia coli, yeast, insect cells, or animal cells.

When using prokaryotes such as Escherichia coli as the host cell, the recombinant vector is preferably a vector that is capable of autonomous replication in prokaryotes and contains a promoter, a ribosome binding sequence, a DNA comprising a portion encoding the human FCRL1, and a transcription termination sequence. Although the recombinant vector does not necessarily have a transcription termination sequence, it is preferred to place the transcription termination sequence immediately below a structural gene. Further, the recombinant vector may contain a gene that controls a promoter.

As the recombinant vector, it is preferred to use a plasmid in which a distance between a Shine-Dalgarno sequence (also called SD sequence), which is a ribosome binding sequence, and an initiation codon is adjusted to an appropriate distance (for example, 6 bases to 18 bases).

As a nucleotide sequence of the DNA encoding the human FCRL 1, bases can be substituted such that the codon is optimal for expression in the host, thereby improving a production rate of the target human FCRL 1.

Any expression vector can be used as long as it can function in a host cell to be used. Examples thereof include pBTrp2, pBTac1, and pBTac2 (manufactured by Roche Diagnostics), pKK233-2 (manufactured by Pharmacia), pSE280 (manufactured by Invitrogen), pGEMEX-1 (manufactured by Promega), pQE-8 (manufactured by Qiagen), pKYP10 (JP58-110600A), pKYP200 [Agricultural Biological Chemistry, 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (manufactured by Stratagene Corporation), pTrs30 [prepared from Escherichia coli JM109/pTrS30 (FERM BP-5407)], pTrs32 [prepared from Escherichia coli JM109/pTrS32 (FERM BP-5408)], pGHA2 [prepared from Escherichia coli IGHA2 (FERM BP-400), JP60-221091A], pGKA2 [prepared from Escherichia coli IGKA2 (FERM BP-6798), JP60-221091A], pTerm2 (US Patent No. 4,686,191, US Patent No. 4,939,094, US Patent No. 160,735), pSupex, pUB110, pTP5, pC194, and pEG400 [J. Bacteriol., 172, 2392 (1990)], pGEX (manufactured by Pharmacia), a pET system (manufactured by Novagen), or pME 18 SFL 3.

Any promoter may be used as long as it can function in a host cell to be used. Examples thereof include a promoter derived from Escherichia coli or a phage, such as a trp promoter (Ptrp), a lac promoter, a PL promoter, a PR promoter, or a T7 promoter Examples thereof include an artificially designed promoter such as a tandem promoter with two Ptrps arranged in series, a tac promoter, a lacT7 promoter, and a let I promoter

Examples of the host cell include Escherichia coli XL 1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, and Escherichia coli DH5α.

Any method for introducing a recombinant vector into a host cell can be used as long as it introduces a DNA into a host cell to be used, such as a method using calcium ions [Proc. Natl. Acad. Sci. USA, 69, 2110 (1972), Gene, 17, 107 (1982), Molecular & General Genetics, 168, 111 (1979)].

When using an animal cell as a host, any expression vector that can function in an animal cell can be used. Examples thereof include pcDNAI, pCDM8 (manufactured by Funakoshi), pAGE107 [JP3-22979A; Cytotechnology, 3, 133 (1990)], pAS3-3 (JP2-227075A), pCDM8 [Nature, 329, 840 (1987)], pcDNAI/Amp (manufactured by Invitrogen), pcDNA3.1 (manufactured by Invitrogen), pREP4 (manufactured by Invitrogen), pAGE103 [J. Biochemistry, 101, 1307 (1987)], and pAGE210, pME18SFL3, pKANTEX93 (WO97/10354), N5KG1val (US Patent. No. 6,001,358), INPEP4 (manufactured by Biogen-IDEC), and a transposon vector (WO2010/143698).

Any promoter can be used as long as it can function in an animal cell, and examples thereof include a cytomegalovirus (CMV) immediate early (IE) gene promoter, a SV40 early promoter, a retrovirus promoter, a metallothionein promoter, a heat shock promoter, an SRα promoter, or a Moloney murine leukemia virus promoter or enhancer. A human CMV IE gene enhancer may be used together with the promoter

Examples of the host cell include human leukemia cells Namalwa cells, monkey cells COS cells, Chinese hamster ovary cells CHO cells [Journal of Experimental Medicine, 108, 945 (1958); Proc. Natl. Acad. Sci. USA, 60 , 1275 (1968); Genetics, 55, 513 (1968); Chromosoma, 41, 129 (1973); Methods in Cell Science, 18, 115 (1996); Radiation Research, 148, 260 (1997); Proc. Natl. Acad. Sci. USA, 77, 4216 (1980); Proc. Natl. Acad. Sci., 60, 1275 (1968); Cell, 6, 121 (1975); Molecular Cell Genetics, Appendix I, II (pp. 883-900)]; CHO cells (CHO/DG44 cells) from which a dihydrofolate reductase gene (hereinafter referred to as dhfr) is deleted [Proc. Natl. Acad. Sci. USA, 77, 4216 (1980)], CHO-K1 (ATCC CCL-61), DUkXB11 (ATCC CCL-9096), Pro-5 (ATCC CCL-1781), CHO-S (Life Technologies, Cat#11619), Pro-3, rat myeloma cells YB2/3HL.P2.G11.16Ag.20 (also called YB2/0), mouse myeloma cells NSO, mouse myeloma cells SP2/0-Ag14, and Syrian hamster cells BHK or HBT5637 (JP63-000299A).

Any method for introducing a recombinant vector into a host cell can be used as long as it introduces a DNA into an animal cell, and examples thereof include an electroporation method [Cytotechnology, 3, 133 (1990)], a calcium phosphate method (JP2-227075A), or a lipofection method [Proc. Natl. Acad. Sci. USA, 84, 7413 (1987)].

The human FCRL1 can be produced by culturing, in a medium, a transformant derived from a microorganism, an animal cell, or the like having a recombinant vector incorporated with the DNA encoding the human FCRL1 obtained as described above, producing and accumulating the human FCRL1 in a culture, and collecting the human FCRL1 from the culture. The transformant can be cultured in a medium according to a general method used for culturing hosts.

When expressed in cells derived from eukaryotes, human FCRL1 with an added sugar or sugar chain can be obtained.

When culturing a microorganism transformed with a recombinant vector using an inducible promoter, an inducer may be added to the medium as necessary. For example, when culturing a microorganism transformed with a recombinant vector using a lac promoter, isopropyl-β-D-thiogalactopyranoside or the like may be added to the medium, and when culturing a microorganism transformed with a recombinant vector using a trp promoter, indole acrylic acid or the like may be added to the medium.

Examples of the medium for culturing the obtained transformant using an animal cell as a host include a generally used RPMI1640 medium [The Journal of the American Medical Association, 199, 519 (1967)], an Eagle's MEM medium [Science, 122, 501 (1952)], a Dulbecco's modified MEM medium [Virology, 8, 396 (1959)], a 199 medium [Proc. Soc. Exp. Biol. Med., 73, 1 (1950)], an Iscove's Modified Dulbecco's Medium (IMDM) medium, and a medium containing fetal bovine serum (FBS). The culture is generally performed for 1 day to 7 days under conditions of pH 6 to 8, 30°C to 40°C, and the presence of 5% CO2. If necessary during culture, an antibiotic such as kanamycin and penicillin may be added to the medium.

As a method for expressing the gene encoding the human FCRL1, in addition to direct expression, for example, a method such as secretory production or fusion protein expression [Molecular Cloning, A Laboratory Manual, Second Edition, Cold Spring Harbor Laboratory Press (1989)] can be used.

Examples of the method for producing the human FCRL 1 include a method for producing human FCRL 1 within a host cell, a method for secreting human FCRL 1 outside a host cell, and a method for producing human FCRL 1 on an outer membrane of a host cell. An appropriate method can be selected by changing a host cell to be used and a structure of human FCRL 1 to be produced.

When human FCRL 1 is produced within a host cell or on an outer membrane of a host cell, human FCRL 1 can be actively secreted outside the host cell by using a method of Paulson et al. [J. Biol. Chem., 264, 17619 (1989)], a method of Rowe et al. [Proc. Natl. Acad. Sci., USA, 86, 8227 (1989), Genes Develop., 4, 1288 (1990)], and a method described in JP05-336963A or WO94/23021. A production amount of human FCRL1 can also be increased using a gene amplification system (JP2-227075A) using a dihydrofolate reductase gene, or the like.

The obtained human FCRL1 can be isolated and purified, for example, as follows. When human FCRL 1 is expressed in a dissolved state in cells, the cells are collected by centrifugation after the end of the culture, suspended in an aqueous buffer solution, and then disrupted with an ultrasonic disintegrator, a French press, a Manton-Gaurin homogenizer, a Dyno mill, or the like to obtain a cell-free extract. From a supernatant obtained by centrifuging the cell-free extract, a purified specimen can be obtained by using a general protein isolation and purification method, that is, a method such as a solvent extraction method, a salting-out method with ammonium sulfate or the like, a desalting method, a precipitation method with an organic solvent, diethylaminoethyl (DEAE)-Sepharose, an anion exchange chromatography method using a resin such as DIAION HPA-75 (manufactured by Mitsubishi Chemical Corporation), a cation exchange chromatography method using a resin such as S-Sepharose FF (manufactured by Pharmacia), a hydrophobic chromatography method using a resin such as butyl sepharose and phenyl sepharose, a gel filtration method using molecular sieves, an affinity chromatography method, a chromatofocusing method, and an electrophoresis method such as isoelectric focusing, alone or in combination.

When human FCRL 1 is expressed in an insoluble form in cells, the cells are collected and disrupted in the same manner as above, and centrifuged to collect an insoluble form of human FCRL 1 as a precipitate fraction. The collected insoluble form of human FCRL1 is solubilized with a protein denaturant. After recovering human FCRL 1 to a normal three-dimensional structure by diluting or dialyzing the solubilized solution, a purified specimen of a polypeptide can be obtained by an isolation and purification method same as that described above.

When human FCRL1 or a derivative such as a glycosylated derivative thereof is secreted extracellularly, the human FCRL 1 or the derivative such as a glycosylated derivative thereof can be collected in a culture supernatant. A soluble fraction can be obtained by processing the culture using a method such as centrifugation same as that described above, and a purified specimen can be obtained from the soluble fraction by using an isolation and purification method same as that described above.

The polypeptide comprising a partial sequence of the amino acid sequence of the human FCRL1 used in the present invention can be produced by a method known to those skilled in the art. Specifically, the polypeptide can be prepared by deleting a part of a DNA encoding the amino acid sequence of human FCRL1 and culturing a transformant introduced with an expression vector comprising the DNA. According to the above method, a polypeptide comprising an amino acid sequence in which one or more amino acids are deleted, substituted, or added in the amino acid sequence of human FCRL1 can be obtained.

The human FCRL1 used in the present invention can also be produced by a chemical synthesis method such as an Fmoc method or a tBoc method. The human FCRL 1 can be produced by chemical synthesis using a peptide synthesizer manufactured by Advanced ChemTech Inc., PerkinElmer, Pharmacia, Protein Technology Instruments, Synthecell-Vega, Perceptiv, or Shimadzu.

### (2) Animal Immunization and Preparation of Antibody-producing Cells for Fusion

Animals such as mice, rats, or hamsters aged 3 weeks to 20 weeks are immunized with the antigen obtained in (1), and antibody-producing cells in spleens, lymph nodes, and peripheral blood of the animals are collected. A mouse FCRL1 knockout mouse can also be used as an immunized animal.

The immunization is performed by administering an antigen into subcutaneous, vein, or abdominal cavity of an animal together with, for example, a suitable adjuvant such as complete Freund's adjuvant or aluminum hydroxide gel and Bordetella pertussis vaccine. When the antigen is a partial peptide, a conjugate with a carrier protein such as a bovine serum albumin (BSA) or a keyhole limpet hemocyanin (KLH) is prepared and used as an immunogen.

After the first administration, the antigen is administered 5 times to 10 times every 1 week to 2 weeks. Blood is collected on day 3 to day 7 after each administration, and an antibody titer of the serum thereof is measured using enzyme immunoassay [Antibodies-A Laboratory Manual, Cold Spring Harbor Laboratory (1988)] or the like. An animal whose serum exhibits a sufficient antibody titer against the antigen used for immunization is used as a supply source of antibody-producing cells for fusion.

Tissues containing antibody-producing cells, such as a spleen, are excised from the immunized animal on day 3 to day 7 after the final administration of the antigen, and the antibody-producing cells are collected. When using spleen cells, the spleen is shredded and loosened, followed by centrifuging, and then erythrocytes are further removed to obtain antibody-producing cells for fusion.

### (3) Preparation of Myeloma Cells

As myeloma cells, an established cell line obtained from a mouse is used, for example, a 8-azaguanine resistant mouse (BALB/c derived) myeloma cell line P3-X63Ag8-U1 (P3-U1) [Current Topics in Microbiology and Immunology, 18, 1 (1978)], P3-NS1/1-Ag41 (NS-1) [European J. Immunology, 6, 511 (1976)], SP2/0-Ag14 (SP-2) [Nature, 276, 269 (1978)], P3-X63-Ag8653 (653) [J. Immunology, 123, 1548 (1979)], andP3-X63-Ag8 (X63) [Nature, 256, 495 (1975)] are used.

The myeloma cells are subcultured in a normal medium [RPMI 1640 medium supplemented with glutamine, 2-mercaptoethanol, gentamycin, FBS, and 8-azaguanine], and subcultured in the normal medium 3 to 4 days before cell fusion to ensure a cell number of 2×10⁷ or more on the day of fusion.

### (4) Cell Fusion and Preparation of Monoclonal Antibody-producing Hybridomas

The antibody-producing cells for fusion obtained in (2) and the myeloma cells obtained in (3) are thoroughly washed with a Minimum Essential Medium (MEM) or PBS (1.83 g of disodium phosphate, 0.21 g of potassium linate, 7.65 g of sodium chloride, 1 liter of distilled water, pH 7.2), and mixed such that the number of cells of antibody-producing cells for fusion:myeloma cells = 5:1 to 10:1, followed by performing centrifugation and then removing a supernatant. After thoroughly loosening a precipitated cell group, a mixed solution of polyethylene glycol-1000 (PEG-1000), a MEM medium, and dimethyl sulfoxide is added thereto at 37°C while stirring. Further, 1 mL to 2 mL of the MEM medium is added several times every 1 minute to 2 minutes, and then the MEM medium is added to make a total amount to 50 mL. After centrifugation, the supernatant is removed. After gently loosening the precipitated cell group, the cells are gently suspended in an HAT medium [normal medium containing hypoxanthine, thymidine, and aminopterin] as antibody-producing cells for fusion. The suspension is cultured at 37°C for 7 days to 14 days in a 5% CO2 incubator

After culturing, a part of the culture supernatant is removed, and a cell group that reacts to an antigen containing the human FCRL 1 and does not react to an antigen not containing the human FCRL 1 is selected using a hybridoma selection method such as a binding assay to be described later. Next, cloning is performed by a limiting dilution method, and hybridomas with stable and strong antibody titers are selected as monoclonal antibody-producing hybridomas.

### (5) Preparation of Purified Monoclonal Antibody

The monoclonal antibody-producing hybridomas obtained in (4) are intraperitoneally injected into mice or nude mice aged 8 weeks to 10 weeks and treated with pristane [intraperitoneally administered with 0.5 mL of 2,6,10,14-tetramethylpentadecane (pristane) and kept for 2 weeks]. The hybridomas turn into ascites cancer in 10 days to 21 days. An ascites fluid is collected from the mice, centrifuged to remove solids, followed by salting out with 40% to 50% ammonium sulfate and purifying using a caprylic acid precipitation method, a DEAE-Sepharose column, a Protein A column, or a gel filtration column, and IgG or IgM fractions are collected and used as purified monoclonal antibodies.

After culturing the monoclonal antibody-producing hybridomas obtained in (4) in an RPMI1640 medium supplemented with 10% FBS, the supernatant is removed by centrifugation, and the resultant is suspended in a Hybridoma SFM medium, followed by culturing for 3 days to 7 days. The obtained cell suspension can be centrifuged, the supernatant can be purified by a protein A column or a protein G column, an IgG fraction can be collected to obtain purified monoclonal antibodies. To the Hybridoma SFM medium, 5% Daigo GF21 can also be added.

Determination of the antibody subclass is performed by enzyme immunoassay using a subclass typing kit. Quantification of a protein amount is calculated by a Lowry method or absorbance at 280 nm.

### (6) Selection of Monoclonal Antibody

Selection of the monoclonal antibody is performed, for example, by measuring the binding property of the antibody to human FCRL1-expressing cells using flow cytometry as described below. The human FCRL1-expressing cells may be any cells as long as the human FCRL 1 is expressed on the cell surface, and examples thereof include human cells, human cell lines, and the forced human FCRL1-expressing cell line obtained in (1).

After the human FCRL 1-expressing cells are dispensed into a plate such as a 96-well plate, a test substance as a first antibody, such as serum, a culture supernatant of hybridoma, or a purified monoclonal antibody, is dispensed and reacted. After the reaction, cells are thoroughly washed with PBS containing 1% to 10% bovine serum albumin (BSA) (hereinafter, referred to as BSA-PBS) or the like, and then an anti-immunoglobulin antibody labeled with a fluorescent reagent or the like as a second antibody is dispensed and reacted. After sufficiently washing with BSA-PBS or the like, a fluorescence amount of the labeled antibody is measured using a flow site meter to select a monoclonal antibody that specifically reacts with the human FCRL 1-expressing cells.

An antibody that binds to human FCRL 1 in competition with the antibody of the present invention can be obtained by adding a test antibody to the above-described measurement system using flow cytometry and causing the antibody to react. That is, by screening an antibody that inhibits the binding between the antibody of the present invention and the human FCRL1 when the test antibody is added, a monoclonal antibody in competition with the antibody of the present invention can be obtained for binding to the amino acid sequence of the human FCRL 1 or a three-dimensional structure thereof.

An antibody that binds to an epitope comprising an epitope to which the monoclonal antibody that binds to the human FCRL 1 of the present invention binds is obtained by identifying an epitope of the antibody obtained by the above-described screening method using a known method, producing a synthetic peptide comprising the identified epitope, or a synthetic peptide that mimics a three-dimensional structure of the epitope, and performing immunization.

Furthermore, an epitope to which the monoclonal antibody that binds to the human FCRL 1 of the present invention binds and an antibody that binds to an epitope same as the epitope are obtained by identifying an epitope of the antibody obtained by the above-described screening method, producing a partial synthetic peptide of the identified epitope, or a synthetic peptide that mimics a three-dimensional structure of the epitope, and performing immunization.

### 2. Preparation of Recombinant Antibody

As an example of producing the recombinant antibody, a method for producing a human chimeric antibody and a humanized antibody are shown below. A recombinant mouse antibody, rat antibody, or rabbit antibody can also be produced by a similar method.

### (1) Construction of Recombinant Antibody Expression Vector

A recombinant antibody expression vector is an animal cell expression vector incorporated with DNAs each encoding a CH and a CL of a human antibody, and can be constructed by cloning DNAs each encoding a CH and a CL of a human antibody into an animal cell expression vector, respectively.

As a C region of the human antibody, a CH and a CL of any human antibody can be used. For example, a CH of γ1 subclass and a CL of κ class of a human antibody are used. A cDNA is used as the DNAs each encoding a CH and a CL of a human antibody, and a chromosomal DNA consisting of exon and intron can also be used. An amino acid residue can be added, inserted, or substituted at a corresponding position by adding, inserting, or substituting a codon encoding an amino acid residue to a DNA encoding a CH or a CL of a human antibody. Any animal cell expression vector can be used as long as it can incorporate and express a gene encoding the C region of the human antibody. For example, pAGE107 [Cytotechnol., 3, 133 (1990)], pAGE103 [J. Biochem., 101, 1307 (1987)], pHSG274 [Gene, 27, 223 (1984)], pKCR [Proc. Natl. Acad. Sci. USA, 78, 1527 (1981)], pSG1bd2-4 [Cytotechnol., 4, 173

(1990)], or pSE1UK1Sed1-3 [Cytotechnol., 13, 79 (1993)] is used. Examples of a promoter and an enhancer for the animal cell expression vector include an SV40 early promoter [J. Biochem., 101, 1307 (1987)], a moloney murine leukemia virus LTR [Biochem. Biophys. Res. Commun., 149, 960 (1987)], or an immunoglobulin H chain promoter [Cell, 41, 479 (1985)], an enhancer [Cell, 33, 717 (1983)].

As the recombinant antibody expression vector, a recombinant antibody expression vector of a type (tandem type) in which an antibody H chain and L chain are present on the same vector [J. Immunol. Methods, 167, 271 (1994)] is used, from the viewpoint of ease of construction of a recombinant antibody expression vector, ease of introduction into animal cells, and a balance of expression levels of the antibody H chain and L chain in animal cells, and a recombinant antibody expression vector of a type in which the antibody H chain and L chain are present on separate vectors can also be used. As a tandem type recombinant antibody expression vector, pKANTEX93 (WO97/10354), pEE18 [Hybridoma, 17, 559 (1998)], and the like are used.

### (2) Acquisition of cDNA Encoding V Region of Antibody Derived from Non-human Animal and Analysis of Amino Acid Sequence

Acquisition of a cDNA encoding a VH and a VL of a non-human antibody and analysis of an amino acid sequence can be performed as follows.

An mRNA is extracted from a non-human antibody-producing hybridoma cell to synthesize a cDNA. The synthesized cDNA is cloned into a vector such as a phage or a plasmid to prepare a cDNA library. From the library, a recombinant phage or a recombinant plasmid comprising the cDNA encoding the VH or the VL is isolated using, as a probe, a DNA encoding a C region portion or a V region portion of a mouse antibody. A total nucleotide sequence of a VH or a VL of the target mouse antibody on the recombinant phage or the recombinant plasmid is determined, and a total amino acid sequence of the VH or the VL is estimated based on the nucleotide sequence.

As a non-human animal for producing non-human antibody-producing hybridoma cells, mice, rats, hamsters, rabbits, and the like are used, and any animal can be used as long as hybridoma cells can be produced.

For preparation of a total RNA from the hybridomas cell, a guanidine thiocyanate method [Methods in Enzymol, 154, 3 (1987)] or a kit such as an RNA easy kit (manufactured by Qiagen) is used.

For preparation of an mRNA from the total RNA, an oligo (dT) immobilized cellulose column method [Molecular Cloning, A Laboratory Manual, Second Edition, Cold Spring Harbor Laboratory Press (1989)] or a kit such as an Oligo-dT30<Super>mRNA Purification (registered trademark) Kit (manufactured by Takara Bio Inc.) is used. The mRNA can also be prepared from the hybridoma cell using a kit such as a Fast Track mRNA Isolation (registered trademark) Kit (manufactured by Invitrogen) or a QuickPrep mRNA Purification (registered trademark) Kit (manufactured by Pharmacia).

For synthesis of the cDNA and preparation of the cDNA library, a known method [Molecular Cloning, A Laboratory Manual, Second Edition, Cold Spring Harbor Laboratory Press (1989), Current Protocols in Molecular Biology. Supplement 1, John Wiley & Sons (1987-1997)], SuperScript Plasmid System for cDNA Synthesis and Plasmid Cloning (manufactured by Invitrogen), or a ZAP-cDNA Synthesis (registered trademark) Kit (manufactured by Stratagene) is used.

When preparing the cDNA library, any vector can be used as long as it can incorporate a cDNA synthesized using an mRNA extracted from a hybridoma cell as a template. For example, ZAP Express [Strategies, 5, 58 (1992)], pBluescript II SK (+) [Nucleic Acids Research, 17, 9494 (1989)], λZAPII (manufactured by Stratagene), λgt10, λgt11 [DNA Cloning: A Practical Approach, I, 49 (1985)], Lambda BlueMid (manufactured by Clontech), λExCell, pT7T3-18U (manufactured by Pharmacia), pcD2 [Mol. Cell. Biol., 3, 280 (1983)], or pUC18 [Gene, 33, 103 (1985)] is used.

Any Escherichia coli that can be used to introduce, express, and maintain the cDNA library constructed using a phage or plasmid vector can be used. For example, XL1-Blue MRF' [Strategies, 5,81 (1992)], C600 [Genetics, 39, 440 (1954)], Y1088, Y1090 [Science, 222, 778 (1983)], NM522 [J. Mol. Biol., 166, 1 (1983)], K802 [J. Mol. Biol., 16, 118 (1966)], or JM105 [Gene, 38, 275 (1985)] is used.

For selection of a clone of the cDNA encoding a VH or a VL of a non-human antibody from the cDNA library, a colony hybridization method using an isotope- or fluorescently labeled probe, a plaque hybridization method [Molecular Cloning, A Laboratory Manual, Second Edition, Cold Spring Harbor Laboratory Press(1989)], or the like is used.

The cDNA encoding the VH or the VL can also be prepared by preparing primers and performing a Polymerase Chain Reaction method [Molecular Cloning, A Laboratory Manual, Second Edition , Cold Spring Harbor Laboratory Press (1989), Current Protocols in Molecular Biology. Supplement 1, John Wiley & Sons (1987-1997)] using a cDNA synthesized from the mRNA or the cDNA library as a template.

After cleaving the selected cDNA with an appropriate restriction enzyme, or the like, cloning into a plasmid such as pBluescript SK(-) (manufactured by Stratagene) is performed, and a nucleotide sequence of the cDNA is determined by a commonly used nucleotide sequence analysis method. As the nucleotide sequence analysis method, for example, an automatic nucleotide sequence analyzer such as ABI PRISM 3700 (manufactured by PE Biosystems) or an A. L. F. DNA sequencer (manufactured by Pharmacia) is used after performing a reaction such as a dideoxy method [Proc. Natl. Acad. Sci. USA, 74, 5463 (1977)].

By estimating complete amino acid sequences of the VH and the VL from the determined nucleotide sequences, respectively, and comparing with complete amino acid sequences of a VH and a VL of a known antibody [Sequences of Proteins of Immunological Interest, US Dept. Health and Human Services (1991)], it is confirmed whether the obtained cDNA encodes the complete amino acid sequence of each VH and VL of the antibody, which includes a secretion signal sequence. Regarding the complete amino acid sequence of each VH and VL of the antibody, which includes a secretion signal sequence, by comparing with complete amino acid sequences of a VH and a VL of a known antibody [Sequences of Proteins of Immunological Interest, US Dept. Health and Human Services (1991)], a length and an N-terminus amino acid sequence of the secretion signal sequence can be estimated, and further, the subgroup to which they belong can be identified. Amino acid sequences of CDRs of the VH and the VL can be found by comparing with amino acid sequences of a VH and a VL of a known antibody [Sequences of Proteins of Immunological Interest, US Dept. Health and Human Services (1991)].

Using the obtained complete amino acid sequences of the VH and the VL, a similarity search by a BLAST method [J. Mol. Biol., 215, 403 (1990)] using any database such as SWISS-PROT or PIR-Protein is performed, and it can be confirmed whether the complete amino acid sequences of the VH and the VL are novel.

### (3) Construction of Human Chimeric Antibody Expression Vector

A human chimeric antibody expression vector can be constructed by cloning the cDNA encoding the VH or the VL of the non-human antibody upstream of each gene encoding a CH or a CL of the human antibody on the recombinant antibody expression vector obtained in (1).

In order to link a 3' end side of the cDNA encoding the VH or the VL of the non-human antibody and a 5' end side of the CH or the CL of a human antibody, cDNAs of a VH and a VL are prepared in such a way that a nucleotide sequence of a linking portion encodes an appropriate amino acid and is an appropriate restriction enzyme recognition sequence. The prepared cDNAs of the VH and the VL are cloned, respectively, upstream of each gene encoding the CH or the CL of the human antibody on the recombinant antibody expression vector obtained in (1) such that they are expressed in an appropriate form, and a human chimeric antibody expression vector is constructed.

The cDNA encoding the VH or the VL of the non-human antibody can be amplified by PCR using a synthetic DNA having recognition sequences for appropriate restriction enzymes at both ends, and the cDNA can be cloned into the recombinant antibody expression vector obtained in (1).

### (4) Construction of cDNA Encoding V Region of Humanized Antibody

A cDNA encoding a VH or a VL of a humanized antibody can be constructed as follows.

An amino acid sequence of an FR of a VH or a VL of a human antibody is selected for grafting an amino acid sequence of CDR of a VH or a VL of a non-human antibody. The selected amino acid sequence of the FR may be any amino acid sequence derived from a human antibody. For example, an amino acid sequence of an FR of a human antibody registered in a database such as a Protein Data Bank, or a common amino acid sequence of each subgroup of an FR of a human antibody [Sequences of Proteins of Immunological Interest, US Dept. Health and Human Services (1991)] is used. In order to prevent a decrease in binding activity of the antibody, an amino acid sequence of an FR having as high a similarity as possible (at least 60% or more) to an amino acid sequence of a VH or a VL of an original antibody is selected.

Next, an amino acid sequence of CDR of the original antibody are grafted to the amino acid sequence of the FR of the VH or the VL of the selected human antibody to design an amino acid sequence of a VH or a VL of a humanized antibody. By converting the designed amino acid sequence into a DNA sequence in consideration of the usage frequency of codons found in the nucleotide sequence of an antibody gene [Sequences of Proteins of Immunological Interest, US Dept. Health and Human Services (1991)], a cDNA sequence encoding an amino acid sequence of a VH or a VL of a humanized antibody is designed.

Based on the designed DNA sequence, several synthetic DNAs having a length of about 100 bases are synthesized, and a PCR reaction is performed using the synthesized DNA. In this case, six synthetic DNAs are preferably designed for each of VH and VL in view of the reaction efficiency in the PCR reaction and the length of DNA that can be synthesized. Furthermore, by introducing recognition sequences of appropriate restriction enzymes into the 5' or 3' ends of the synthetic DNA located at both ends, a cDNA encoding the VH or the VL of the humanized antibody can be easily cloned into the recombinant antibody expression vector obtained in (1).

After the PCR reaction, each amplified product was cloned into a plasmid such as pBluescript SK(-) (manufactured by Stratagene), and a nucleotide sequence is determined by a method same as that described in (2) to obtain a plasmid having a DNA sequence encoding an amino acid sequence of a VH or a VL of a desired humanized antibody.

Based on the designed DNA sequence, a DNA synthesized using each of the full length of the VH and the full length of the VL as one long chain DNA can be used instead of the PCR amplification product. Furthermore, by introducing the recognition sequence for an appropriate restriction enzyme at both ends of the synthetic long chain DNA, a cDNA encoding the VH or the VL of the humanized antibody can be easily cloned into the recombinant antibody expression vector obtained in (1).

### (5) Modification of Amino Acid Sequence of V Region of Humanized Antibody

When only CDRs of a VH and a VL of a non-human antibody are grafted onto the FRs of the VH and the VL of the human antibody, the antigen binding activity of the humanized antibody is lower than that of the original non-human antibody [BIO/TECHNOLOGY, 9, 266 (1991)]. In the humanized antibody, by identifying, among the amino acid sequences of the FRs of the VH and the VL of the human antibody, an amino acid residue directly associated with binding to an antigen, an amino acid residue interacting with an amino acid residue of CDR, and an amino acid residue maintaining a three-dimensional structure of an antibody and indirectly associated with binding to an antigen, and substituting those amino acid residues with amino acid residues of the original non-human antibody, the reduced antigen binding activity can be increased.

In order to identify the FR amino acid residues associated with the antigen binding activity, the three-dimensional structure of the antibody can be constructed and analyzed by using X-ray crystal analysis [J. Mol. Biol., 112, 535 (1977)], computer modeling [Protein Engineering, 7, 1501 (1994)], or the like. A humanized antibody having a necessary antigen binding activity can be obtained by preparing several types of variants of each antibody, repeatedly examining each correlation with the antigen binding activity, and performing trial and error.

The amino acid residues of the FRs of the VH and the VL of the human antibody can be modified by performing the PCR reaction described in (4) using a synthetic DNA for modification. The nucleotide sequence of the amplified product after the PCR reaction is determined by the method described in (2) to confirm that the desired modification is made.

### (6) Construction of Humanized Antibody Expression Vector

By cloning the cDNA encoding the VH or the VL of the constructed recombinant antibody upstream of the gene encoding the CH or the CL of the human antibody in the recombinant antibody expression vector obtained in (1), a humanized antibody expression vector can be constructed.

For example, by introducing recognition sequences for appropriate restriction enzymes to the 5' or 3' ends of the synthetic DNA used in constructing the VH and the VL of the humanized antibody obtained in (4) and (5) at both ends, respectively, the synthetic DNA is cloned upstream of the gene encoding the CH or the CL of the human antibody in the recombinant antibody expression vector obtained in (1) such that the synthetic DNA is expressed in an appropriate form.

### (7) Transient Expression of Recombinant Antibody

Using the recombinant antibody expression vector obtained in (3) and (6) or an expression vector modified therefrom, transient expression of a recombinant antibody can be performed, and the antigen binding activity of produced various types of human chimeric antibody and humanized antibody can be efficiently evaluated.

Any host cell that can express a recombinant antibody can be used as the host cell into which the expression vector is to be introduced, and for example, COS-7 cells [American Type Culture Collection (ATCC) number: CRL 1651] are used [Methods in Nucleic Acids Res., CRC press, 283 (1991)].

For introduction of the expression vector into COS-7 cells, a DEAE-dextran method [Methods in Nucleic Acids Res., CRC press(1991)], or a lipofection method [Proc. Natl. Acad. Sci. USA, 84, 7413(1987)] is used.

After introduction of the expression vector, an expression level and an antigen binding activity of the recombinant antibody in a culture supernatant are measured using enzyme immunoassay [Monoclonal Antibodies-Principles and practice, Third edition, Academic Press (1996), Antibodies-A Laboratory Manual, Cold Spring Harbor Laboratory (1988), Monoclonal Antibody Laboratory Manual, Kodansha Scientific (1987)], and the like.

### (8) Acquisition of Transformant Stably Expressing Recombinant Antibody and Preparation of Recombinant Antibody

By introducing the recombinant antibody expression vectors obtained in (3), and (6) into appropriate host cells, transformants that stably express the recombinant antibody can be obtained. An electroporation method [JP2-257891A, Cytotechnology, 3, 133 (1990)] is used to introduce the expression vector into a host cell.

Any host cell that can express the recombinant antibody can be used as the host cell into which the recombinant antibody expression vector is to be introduced. For example, CHO-K1 (ATCC CCL-61), DUKXB11 (ATCC CCL-9096), Pro-5 (ATCC CCL-1781), CHO-S (Life Technologies, Cat#11619), rat myeloma cells YB2/3HL.P2. G11.16Ag. 20 (ATCC No.: CRL1662, or also referred to as YB2/0), mouse myeloma cells NS0, mouse myeloma cells SP2/0-Ag14 (ATCC No.: CRL1581), mouse P3X63-Ag8.653 cells (ATCC No.: CRL1580), and CHO cells (CHO/DG44 cells) from which a dihydrofolate reductase gene (hereinafter referred to as dhfr) is deleted [Proc. Natl. Acad. Sci. USA, 77, 4216 (1980)] are used.

A host cell in which an activity of a protein such as an enzyme associated with synthesis of intracellular sugar nucleotide GDP-fucose, a protein such as an enzyme associated with sugar chain modification in which 1-position of fucose α-binds to 6-position of N-acetylglucosamine at a reducing end of an N-glycoside-linked complex sugar chain, a protein associated with transport of intracellular sugar nucleotide GDP-fucose to a Golgi body is reduced or deleted, for example, CHO cells from which an α1,6-fucosyltransferase gene is deleted (WO2005/035586, WO02/31140), or Lecl3 with acquired lectin resistance [Somatic Cell and Molecular genetics, 12, 55 (1986)] can also be used.

After introduction of the expression vector, a transformant that stably expresses the recombinant antibody is selected by performing culture in an animal cell culture medium containing a drug such as G418 sulfate (hereinafter referred to as G418) (JP2-257891A).

Examples of the animal cell culture medium include a RPMI 1640 medium (manufactured by Invitrogen), a GIT medium (manufactured by Japan Pharmaceutical Co., Ltd.), an EX-CELL 301 medium (manufactured by JRH Co., Ltd.), an IMDM medium (manufactured by Invitrogen), a Hybridoma-SFM medium (manufactured by Invitrogen), and a medium supplemented with various additives such as FBS. The obtained transformant is cultured in a medium to express and accumulate the recombinant antibody in the culture supernatant. An expression level and an antigen binding activity of the recombinant antibody in the culture supernatant can be measured by ELISA or the like. The expression level of the recombinant antibody produced by the transformant can be increased by using a dhfr amplification system (JP2-257891A), or the like.

The recombinant antibody can be purified from the culture supernatant of the transformant using a protein A column [Monoclonal Antibodies-Principles and practice, Third edition, Academic Press (1996), Antibodies-A Laboratory Manual, Cold Spring Harbor Laboratory (1988)]. Alternatively, methods used for protein purification, such as gel filtration, ion exchange chromatography, and ultrafiltration can be combined.

A molecular weight of the H chain, the L chain, or the entire antibody molecule of the purified recombinant antibody can be measured using polyacrylamide gel electrophoresis [Nature, 227, 680 (1970)], Western blotting [Monoclonal Antibodies-Principles and practice, Third edition, Academic Press (1996), Antibodies-A Laboratory Manual, Cold Spring Harbor Laboratory (1988)], or the like.

### 3. Activity Evaluation of Purified Monoclonal Antibody or Antibody Fragment Thereof

The activity of the purified monoclonal antibody or the antibody fragment thereof of the present invention can be evaluated as follows.

The binding activity of the antibody or the antibody fragment thereof of the present invention to the human FCRL1 is measured using the flow cytometry described in the above-described 1-(6). The measurement can be performed using a fluorescent antibody method [Cancer Immunol. Immunother., 36, 373 (1993)] or the like.

A CDC activity or an ADCC activity for human FCRL1-expressing cells can be measured by a known measurement method [Cancer Immunol. Immunother., 36, 373(1993); Current protocols in Immunology, Chapter7. Immunologic studies in humans, Editor, John E, Coligan et al., John Wiley & Sons, Inc.,(1993)].

### 4. Method for Controlling Effector Activity of Antibody

As a method for controlling an effector activity of the monoclonal antibody of the present invention, there has been known a method for controlling an amount of fucose (also called core fucose) that alpha-1,6-binds to N-acetylglucosamine (GlcNAc) present at a reducing end of an N-linked complex-type sugar chain that binds to asparagine (Asn) at position 297 in an Fc region of an antibody (WO2005/035586, WO2002/31140, and WO00/61739), and a method for controlling by modifying amino acid residues in an Fc region of an antibody. The effector activity of the monoclonal antibody of the present invention can be controlled using any method.

The effector activity refers to an antibody-dependent activity caused through an Fc region of an antibody, and known examples thereof include an ADCC activity, a CDC activity, and an antibody-dependent phagocytosis activity (ADP activity) based on phagocytes such as macrophages or dendritic cells.

As a method for measuring the effector activity, for example, target inflammatory cells, human peripheral blood mononuclear cells (PBMC) as an effector, and inflammatory cell-specific antibodies are mixed and incubated for about 4 hours, and then a released lactate dehydrogenase (LDH) is measured as an indicator of cell damage. Alternatively, after adding an antibody that recognizes a blood cell-specific antigen such as CD20 to human whole blood and performing incubation, a decrease in the number of target blood cells can be measured as the effector activity. For example, after mixing human whole blood with another target cell, further adding an antibody specific to the target cell, and performing incubation, a decrease in the number of target cells can be measured as the effector activity. In either case, the effector activity can be measured by a free LDH method, a free 51Cr method, a flow cytometry method, or the like.

By controlling a content of core fucose of an N-linked complex sugar chain of an Fc of an antibody, the effector activity of the antibody can be increased or decreased. As a method for decreasing a content of fucose that binds to an N-linked complex sugar chain that binds to an Fc of an antibody, an antibody to which no fucose binds can be obtained by expressing the antibody using CHO cells from which an α1,6-fucosyltransferase gene is deleted. An antibody to which no fucose binds has a high ADCC activity.

On the other hand, as a method for increasing the content of fucose that binds to an N-linked complex sugar chain that binds to an Fc of an antibody, an antibody to which fucose binds can be obtained by expressing the antibody using a host cell introduced with an α1, 6-fucose transferase genetic gene. An antibody to which fucose binds has an ADCC activity lower than that of the antibody to which no fucose binds.

The ADCC activity or the CDC activity can be increased or decreased by modifying the amino acid residue of the Fc region of the antibody. For example, the CDC activity of the antibody can be increased by using the amino acid sequence of the Fc region described in US Patent Application Publication No. 2007/0148165.

The ADCC activity or the CDC activity can be increased or decreased by performing an amino acid modification described in US Patent No. 6,737,056, US Patent No. 7,297,775, or US Patent No. 7,317,091. The antibody of the present invention also includes an antibody whose blood half-life is controlled by controlling a reactivity to an Fc receptor by performing an amino acid modification or sugar chain modification in the antibody constant region described above, for example, an amino acid modification described in JP2013-165716A or JP2012-021004A.

Furthermore, by using the above-described methods in combination for one antibody, an antibody whose effector activity and blood half-life are controlled can be obtained.

### 5. Method for Producing Antibody-drug Conjugate Containing Anti-FCRL1 Monoclonal Antibody or Antibody Fragment Thereof of Present Invention

The antibody-drug conjugate containing the anti-FCRL1 monoclonal antibody or the antibody fragment thereof of the present invention can be produced by binding the monoclonal antibody and a drug using a chemical, enzymatic, or genetic engineering method.

### (1) Method for Linking Monoclonal Antibody and Drug by Chemical Method

A reactive substituent is introduced into the antibody by adding, inserting, or substituting an amino acid residue having an appropriate substituent at any position by the method described in 2.(1). A bond of the antibody at any position may be cleaved by reduction, hydrolysis, enzymatic decomposition, or the like to form a reactive substituent. Further, a sugar having a reactive substituent can be introduced into a sugar chain contained in the antibody molecule by using an enzyme such as glycosidase or a sugar transferase. Examples of such a reactive substituent include an α, β-unsaturated carbonyl group, an α, β-unsaturated sulfinyl group, an α, β-unsaturated sulfonyl group, a thiol group, an amino group, an amide group, a formyl group, a carboxyl group, an azide group, an alkynyl group, an alkenyl group, a haloalkyl group, and a carbonyl group.

A chemical structure capable of reacting with the reactive functional group introduced into the antibody is introduced into a drug or linker, and the antibody and the drug or linker are bonded by reacting under appropriate reaction conditions. The linker may be bound to the drug before reacting with the antibody, or may be bound to the drug after reacting with the antibody. The linker and the drug can be bound to each other by a known method (for example, a method described in S. J. Walsh et al. Chem. Soc. Rev. 2021, 50, 1305-1353; Tumey, L. Nathan (2020). Antibody-Drug Conjugates-Methods and Protocols: New York, Springer; and Laurent Ducry (2013). Antibody-Drug Conjugate: New York, Springer).

### (2) Method for Linking Monoclonal Antibody and Drug by Enzymatic Method

For example, an amino acid sequence recognized by a specific enzyme is added or substituted at the C-terminal of the antibody by the method described in 2.(1). Examples of such an amino acid sequence include a CaaX tag (where C represents cysteine, a represents an any aliphatic amino acid, and X represents a C-terminal amino acid) which are recognized by, for example, farnesyltransferase and geranyltransferase.

A functional group that is transferred by an enzyme that recognizes the amino acid sequence introduced into the antibody is introduced into the drug or linker, and the antibody and the drug or linker are bound by an enzymatic reaction with the above-described amino acid sequence under appropriate conditions. Examples of the functional group corresponding to the above-described CaaX tag include a prenyl group such as a geranyl group and a farnesyl group. The linker may be bound to the drug before reacting with the antibody, or may be bound to the drug after reacting with the antibody. The linker and the drug can be bound by a known method.

### (3) Method for Linking Monoclonal Antibody and Drug by Genetic Engineering Method

When the drug is a protein or peptide, the monoclonal antibody and the drug can be bound by designing a DNA encoding the protein or peptide, adding, inserting, or substituting the DNA to an antibody gene at any position, and expressing the DNA in the same manner as in 2.

### 6. Therapeutic Method for Disease Using Anti-human FCRL1 Monoclonal Antibody or Antibody Fragment Thereof of Present Invention

The monoclonal antibody or the antibody fragment thereof of the present invention can be used to treat any disease associated with human FCRL 1 as long as it expresses FCRL 1.

The therapeutic agent containing the monoclonal antibody or the antibody fragment thereof of the present invention may contain only the antibody or the antibody fragment thereof as an active ingredient, and the therapeutic agent can be mixed together with one or more pharmacologically acceptable carriers and provided as a pharmaceutical formulation prepared by a method known in the pharmaceutical art.

Examples of the administration route include oral administration, and parenteral administration such as buccal, tracheobronchial, intrarectal, subcutaneous, intramuscular, or intravenous administration. Examples of a dosage form include sprays, capsules, tablets, powders, granules, syrups, emulsions, suppositories, injections, ointments, and tapes.

Examples of the preparation suitable for oral administration include an emulsion, a syrup, a capsule, a tablet, a powder, and a granule.

A liquid preparation such as an emulsion or a syrup can be produced using, as an additive, water, sugars such as sucrose, sorbitol or fructose, glycols such as polyethylene glycol or propylene glycol, oils such as sesame oil, olive oil, or soybean oil, antiseptics such as a p-hydroxybenzoic acid ester, or flavors such as strawberry flavor or peppermint.

The capsule, the tablet, the powder, the granule, and the like can be produced using, as an additive, an excipient such as lactose, glucose, sucrose, or mannitol, a disintegrator such as starch or sodium alginate, a lubricant such as magnesium stearate or talc, a binder such as polyvinyl alcohol, hydroxypropyl cellulose, or gelatin, a surfactant such as a fatty acid ester, or a plasticizer such as glycerin.

Examples of the preparation suitable for parenteral administration include an injection, suppository, and a spray. The injection can be produced using a salt solution, a glucose solution, or a carrier formed of a mixture of both. The suppository can be produced using a carrier such as cacao butter, hydrogenated aliphatic, or carboxylic acid.

The spray can be produced using a carrier that does not stimulate the oral cavity and the respiratory tract mucosa of a recipient and that allows the monoclonal antibody and the antibody fragment thereof of the present invention to be dispersed as fine particles and facilitate absorption. Examples of the carrier include lactose and glycerin. The preparation can also be produced as an aerosol or a dry powder. Further, a component shown as an additive in an appropriate preparation for oral administration can also be added to the above parental agent.

### 7. Diagnostic Method for Disease Using Anti-human FCRL 1 Monoclonal Antibody or Antibody Fragment thereof of Present Invention

The disease associated with human FCRL1 can be diagnosed by detecting or measuring human FCRL1 or a cell expressing the human FCRL 1 using the monoclonal antibody, the antibody fragment thereof, or the antibody-drug conjugate of the present invention.

Diagnosis of a cancer disease, an autoimmune disease, and an inflammatory disease, which are a disease associated with human FCRL1, can be performed, for example, by detecting or measuring human FCRL1 present in the patient body by an immunological method. The diagnosis can be performed by detecting human FCRL 1 expressed in cells in the patient body using an immunological method such as flow cytometry.

The immunological technique is a method of detecting and measuring an antibody amount or an antigen amount by using an antigen or an antibody subjected to labeling. Examples thereof include a radiolabeled immunoantibody method, enzyme immunoassay, fluorescence immunoassay, luminescent immunoassay, Western blotting, or a physicochemical method.

In the radiolabeled immunoantibody method, for example, an antigen, a cell expressing an antigen, or the like is allowed to react with the antibody or the antibody fragment thereof of the present invention, followed by further allowing to react with a radiolabeled anti-immunoglobulin antibody or an antibody fragment thereof, and then measurement is performed using a scintillation counter or the like.

In the enzyme immunoassay, for example, an antigen, a cell expressing an antigen, or the like is allowed to react with the antibody or the antibody fragment thereof of the present invention, followed by further allowing to react with an anti-immunoglobulin antibody and a binding fragment labeled with an enzyme, or the like, and then a substrate is added and an absorbance of the reaction solution is measured using an absorptiometer. For example, sandwich ELISA is used. As a labeling material to be used in the enzyme immunoassay method, a known enzyme label [Enzyme Immunoassay, Igaku Shoin (1987)] can be used.

For example, an alkaline phosphatase label, a peroxidase label, a luciferase label, or a biotin label is used. The sandwich ELISA is a method in which an antibody is conjugated to a solid phase, then an antigen to be detected or measured is trapped, and a second antibody is allowed to react with the trapped antigen. In the ELISA, two types of antibodies or antibody fragments that recognize the antigen to be detected or measured and have different antigen recognition sites are prepared, and among them, a first antibody or an antibody fragment thereof is adsorbed onto a plate (for example, a 96-well plate) in advance, and then a second antibody or an antibody fragment thereof is labeled with a fluorescent substance such as FITC, an enzyme such as peroxidase, or biotin. Cells isolated from a living body or a lysate thereof, tissues or a lysate thereof, cell culture supernatant, serum, a pleural fluid, an ascites fluid, or an eye fluid is allowed to react with the plate to which the above antibody is adsorbed, followed by allowing to react with a labeled monoclonal antibody or an antibody fragment to perform a detection reaction depending on the labeling substance. An antigen concentration in a test sample is calculated from a calibration curve prepared by stepwise diluting a known antigen. As the antibody used in the sandwich ELISA method, either a polyclonal antibody or a monoclonal antibody may be used, and an antibody fragment such as Fab, Fab', or F(ab') 2 may be used. The combination of two types of antibodies used in the sandwich ELISA may be a combination of monoclonal antibodies that recognize different epitopes and antibody fragments thereof, or a combination of a polyclonal antibody and a monoclonal antibody or an antibody fragment thereof.

For the fluorescence immunoassay, for example, a method described in the literature [Monoclonal Antibodies-Principles and practice, Third edition, Academic Press (1996), Monoclonal Antibody Experiment Manual, Kodansha Scientific (1987)] is used. As the labeling material used in the fluorescence immunoassay, a known fluorescent label [Fluorescent Antibody Method, Soft Science Co., Ltd. (1983)] can be used. For example, FITC or RITC is used.

For the luminescent immunoassay, for example, the measurement is performed by a method described in, for example, the literature [Bioluminescence, Chemiluminescence Clinical Examination 42, Hirokawa Shoten (1998)]. Examples of the labeling material used in the luminescent immunoassay include a known luminescent label. Acridinium ester or lophine is used.

For the Western blotting, after fractionating antigens or cells expressing the antigens using SDS (sodium dodecyl sulfate)-PAGE (polyacrylamide gel) [Antibodies-A Laboratory Manual Cold Spring Harbor Laboratory (1988)], the gel is blotted onto a polyvinylidene difluoride (PVDF) membrane or a nitrocellulose membrane, the membrane is allowed to react with an antibody or an antibody fragment thereof that recognizes the antigen, followed by further allowing to react with an anti-mouse IgG antibody or a binding fragment labeled with a fluorescent substance such as FITC, an enzyme label such as peroxidase, or a biotin label, and then measurement is performed by visualizing the label.

An example is shown below. Cells or tissues expressing a polypeptide comprising the amino acid sequence represented by SEQ ID NO: 3 or 4 are lysed, and 0.1 µg to 30 µg of protein per lane is migrated by SDS-PAGE under reducing conditions. The migrated proteins are transferred to a PVDF membrane and allowed to react with PBS containing 1% to 10% BSA (hereinafter referred to as BSA-PBS) at room temperature for 30 minutes to perform a blocking operation. Here, the resultant is allowed to react with the monoclonal antibody of the present invention, followed by washing with PBS containing 0.05% to 0.1% Tween-20 (hereinafter, referred to as Tween-PBS) and allowing to react with peroxidase-labeled goat anti-mouse IgG at room temperature for 2 hours. The polypeptide comprising the amino acid sequence represented by SEQ ID NO: 3 or 4 is detected by washing with Tween-PBS and detecting a band to which the monoclonal antibody is bound using ECL Western Blotting Detection Reagents (manufactured by Amersham plc) or the like. As the antibody used for detection in the Western blotting, an antibody capable of binding to a polypeptide not retaining a natural three-dimensional structure is used.

The physicochemical method is performed, for example, by binding human FCRL1 as an antigen to the monoclonal antibody of the present invention or the antibody fragment thereof to form an aggregate and detecting the aggregate. As the physicochemical method, a capillary method, a single radial immunodiffusion method, turbidimetric inhibition immunoassay, or latex turbidimetric inhibition immunoassay [Clinical Testing Projection, Kanehara (1998)] can also be used. In the latex turbidimetric inhibition immunoassay, when a carrier such as polystyrene latex with a particle size of about 0.1 µm to 1 µm that is sensitized with an antibody or antigen is used and an antigen-antibody reaction is caused by the corresponding antigen or antibody, scattered light in the reaction solution increases and transmitted light decreases. By detecting this change as absorbance or integrating sphere turbidity, the antigen concentration in the test sample is measured.

Known immunological detection methods can be used to detect or measure cells expressing human FCRL1, and among them, an immunoprecipitation method, an immunocytostaining method, an immunohistological staining method, a fluorescent antibody staining method, or the like is preferably used.

In the immunoprecipitation method, cells expressing human FCRL1, or the like are allowed to react with the monoclonal antibody or the antibody fragment thereof of the present invention, and then a carrier having a binding ability specific to immunoglobulin, such as Protein G-Sepharose, is added to precipitate an antigen-antibody complex. The immunoprecipitation method can also be performed by the following method. The monoclonal antibody or the antibody fragment thereof of the present invention is immobilized on a 96-well plate for ELISA, and then blocked with BSA-PBS. When the antibody is in an unpurified state, such as in a hybridoma culture supernatant, anti-mouse immunoglobulin, anti-rat immunoglobulin, protein-A, protein-G, or the like is immobilized in advance on an ELISA 96-well plate, and blocked with BSA-PBS, and then the hybridoma culture supernatant is dispensed and bound. Next, after discarding the BSA-PBS and thoroughly washing with PBS, a lysate of cells or tissues expressing human FCRL 1 is allowed to react. After thoroughly washing the plate, the immunoprecipitate is extracted with a SDS-PAGE sample buffer and detected by Western blotting as described above.

The immunocytostaining method or immunohistological staining method is a method in which cells or tissues expressing antigens are treated with a surfactant, methanol, or the like to improve antibody permeability according to the case, allowed to react with the monoclonal antibody of the present invention, and further allowed to react with an anti-immunoglobulin antibody or binding fragment thereof labeled with a fluorescent label such as FITC, an enzyme label such as peroxidase, or a biotin label, and then the label is visualized and viewed under a microscope. Detection can be performed using a fluorescent antibody staining method in which cells are allowed to react with a fluorescently labeled antibody and analyzed using a flow cytometer [Monoclonal Antibodies-Principles and practice, Third edition, Academic Press (1996), Monoclonal Antibody Experiment Manual, Kodansha Scientific (1987)]. In particular, cells expressing the monoclonal antibody or the antibody fragment thereof of the present invention that binds to human FCRL1 while retaining a natural three-dimensional structure can be detected by fluorescent antibody staining.

In the fluorescent antibody staining method, when using an FMAT 8100 HTS system (manufactured by Applied Biosystems) or the like, an antigen amount and an antibody amount can be measured without separating the formed antibody-antigen complex and a free antibody or antigen not involved in the formation of the antibody-antigen complex.

### 8. Therapeutic Method for Disease Using Anti-human FCRL1 Monoclonal Antibody or Antibody Fragment Thereof of Present Invention

The monoclonal antibody, the antibody fragment thereof, or the antibody-drug conjugate of the present invention can be used for treating any disease associated with human FCRL 1 as long as the disease is associated with human FCRL1.

The therapeutic agent containing the monoclonal antibody or the antibody fragment thereof of the present invention may contain only the antibody or the antibody fragment thereof as an active ingredient, and the therapeutic agent can be mixed together with one or more pharmacologically acceptable carriers and provided as a pharmaceutical formulation prepared by a method known in the pharmaceutical art.

Examples of the administration route include oral administration, and parenteral administration such as buccal, tracheobronchial, intrarectal, subcutaneous, intramuscular, or intravenous administration. Examples of a dosage form include sprays, capsules, tablets, powders, granules, syrups, emulsions, suppositories, injections, ointments, and tapes.

Examples of the preparation suitable for oral administration include an emulsion, a syrup, a capsule, a tablet, a powder, and a granule.

A liquid preparation such as an emulsion or a syrup can be produced using, as an additive, water, sugars such as sucrose, sorbitol or fructose, glycols such as polyethylene glycol or propylene glycol, oils such as sesame oil, olive oil, or soybean oil, antiseptics such as a p-hydroxybenzoic acid ester, or flavors such as strawberry flavor or peppermint.

The capsule, the tablet, the powder, the granule, and the like can be produced using, as an additive, an excipient such as lactose, glucose, sucrose, or mannitol, a disintegrator such as starch or sodium alginate, a lubricant such as magnesium stearate or talc, a binder such as polyvinyl alcohol, hydroxypropyl cellulose, or gelatin, a surfactant such as a fatty acid ester, or a plasticizer such as glycerin.

Examples of the preparation suitable for parenteral administration include an injection, suppository, and a spray. The injection can be produced using a salt solution, a glucose solution, or a carrier formed of a mixture of both. The suppository can be produced using a carrier such as cacao butter, hydrogenated aliphatic, or carboxylic acid.

The spray can be produced using a carrier that does not stimulate the oral cavity and the respiratory tract mucosa of a recipient and that allows the monoclonal antibody and the antibody fragment thereof of the present invention to be dispersed as fine particles and facilitate absorption. Examples of the carrier include lactose and glycerin. The preparation can also be produced as an aerosol or a dry powder. Further, a component shown as an additive in an appropriate preparation for oral administration can also be added to the above parental agent.

Hereinafter, the present invention will be specifically explained with reference to Examples, but the present invention is not limited to the following Examples.

### [Example]

### [Example 1] Preparation of Known Anti-FCRL 1 Chimeric Antibody

A chimeric antibody was prepared based on amino acid sequence information on variable regions of known E3 and E9 (Blood., 2008, 111, 338-43), 1F9, and 2A10 (WO2005/063299), and 7G8, 2G5, and 5A2 (WO2005/097185). Amino acid sequences of a heavy chain variable region (VH) and a light chain variable region (VL) of each antibody are shown in Table 1.

**[Table 1]**

| Clone name | VH | VL |
|---|---|---|
| E3 | SEQ ID NO: 5 | SEQ ID NO: 6 |
| E9 | SEQ ID NO: 7 | SEQ ID NO: 8 |
| 1F9 | SEQ ID NO: 9 | SEQ ID NO: 10 |
| 2A10 | SEQ ID NO: 11 | SEQ ID NO: 12 |
| 7G8 | SEQ ID NO: 13 | SEQ ID NO: 14 |
| 2G5 | SEQ ID NO: 15 | SEQ ID NO: 16 |
| 5A2 | SEQ ID NO: 17 | SEQ ID NO: 18 |

An expression vector of the chimeric antibody was constructed by inserting a VH region into a pFUSE-CHIg-hG1 plasmid vector and inserting a VL region into a pFUSE2-CLIg-hk plasmid vector. A vector was used in which serine at position 239 (EU numbering) in a constant region of a heavy chain was converted to cysteine. A human chimeric antibody was produced using these vectors and an Expi293 Expression System (Life Technologies). The procedure was performed as follows according to the attached manual.

7.5 × 10⁸ Expi293F cells (Thermo Fisher Scientific) per reaction were added to 255 mL of Expi293 Expression Medium (Thermo Fisher Scientific). 200 µg of pFUSE-CHIg-hG1 plasmid vector, 100 µg of pFUSE2-CLIg-hk plasmid vector, and an ExpiFectamin 293 Reagent (Thermo Fisher Scientific) were added to Opti-MEM (Thermo Fisher Scientific), followed by allowing to stand for 20 minutes, and then the plasmid solution was added to the above-described cell-containing solution. After further overnight culture, an ExpiFectamine 293 Transfection Enhancer was added to the cell-containing solution (a total culture volume was 300 mL). The cell-containing solution was further cultured for 2 days, and then the culture supernatant was collected.

For the purification of the antibody, MabSelect SuRe (GE Healthcare) was used. The collected culture supernatant was centrifuged, and the obtained culture supernatant was filtered through a filter. A column was filled with 1 mL of carrier, and a buffer was replaced with DPBS. The culture supernatant was added to the column, the antibody was adsorbed to the carrier, and the column was washed twice with 10 mL of DPBS. To the column, 2.5 mL of Arg-Antibody Elution Buffer (Nacalai Tesque) was added to elute the antibody. The antibody solution was desalted using a NAP column (GE Healthcare) and used for subsequent analysis.

The obtained antibody is an IgG1 antibody in which serine at position 239 (EU numbering) in the heavy chain is substituted with cysteine (hereinafter, also referred to as S239C mutation). A heavy chain constant region comprising the S239C mutation comprises the amino acid sequence represented by SEQ ID NO: 80. When E3, 2G5, and 5A2 were prepared, aggregates were detected, so that they were not subjected to ADC conversion.

### [Example 2] Preparation of ADC of Known Anti-FCRL 1 Chimeric Antibody

An ADC can be produced by a method described in Bioconjug Chem 2013, 24(7), 1256-1263. The ADC was prepared by reacting the FCRL1 chimeric antibody having the S239C mutation and prepared in Example 1 with SG3249, which is a PBD dimer payload linker (Med. Chem. Lett. 2016, 7, 983-987).

Analysis of a drug-to-antibody ratio (DAR) can be performed by using a high-performance liquid chromatography device and a reverse phase column (reverse phase HPLC) after converting the ADC into a light chain fragment and a heavy chain fragment by pretreatment with a reducing agent. The DAR is calculated from peak area ratios of an unreacted light chain, a drug-bound light chain, an unreacted heavy chain, and a drug-bound heavy chain. The drug-to-antibody ratios of all prepared ADCs were 1.8 to 1.9. 2A10 was excluded from the evaluation because of the difficulty in producing an ADC with controlled DAR.

For a DNP antibody produced according to the method described in Example 1 using a vector encoding an anti-2,4-dinitrophenol (DNP) IgG1 antibody (S239C mutation) described in Clin Cancer Res 2005, 11(8), 3126-3135, ADCs (anti-DNP antibody-ADCs) were produced by the same method and used as negative controls in the following studies.

### [Example 3] In Vivo Drug Efficacy Evaluation of ADC of Known Anti-FCRL 1 Chimeric Antibody

SU-DHL-6 cells suspended in Phosphate Buffered Saline (PBS) containing 50 vol% Matrigel (Corning) were subcutaneously grafted into a ventral side of male SCID mice aged 5 weeks at 1 × 10⁷ cells/0.1 mL/head. On day 21 after grafting, individuals having a tumor volume of 120 mm³ or more were selected and grouped.

The day of grouping was set as day 0, and on day 0, a diluted known FCRL 1 chimeric antibody-ADC of 0.3 mg/kg body weight or an anti-DNP antibody-ADC of 0.4 mg/kg body weight was administered into the tail vein. A composition of a Vehicle is 10 mmol/L L-sodium glutamate, 262 mmol/L D-sorbitol, 0.05 mg/mL polysorbate 80, and pH 5.5. A tumor volume and weight of the mouse were measured twice a week. The results are shown in FIG. 1. The ADC of the known anti-human FCRL1 chimeric antibody was the ADC prepared in Example 2.

As shown in FIG. 1, among the ADC of the known anti-human FCRL 1 antibody, 7G8-ADC exhibited the strongest anti-tumor activity. In the case of a Ramos cell subcutaneous grafted mouse model, 7G8-ADC also exhibited the strongest antitumor activity (details are omitted).

### [Example 4] Acquisition of Novel Anti-human FCRL 1 Mouse Antibody

As an immune host, A/J, BALB/c, or C57BL6 mice (all are Japan SLC) were used, and as an immunogen, a plasmid vector (15 µg to 50 µg) expressing a full length of human FCRL1 (NP_443170.1), a full length of cynomolgus monkey FCRL1 (XP_015310712.1), or a mutant thereof, a fusion protein (10 µg to 25 µg) of a human FCRL1 extracellular domain and a C-terminal His Tag, or Rabbit IgG1-Fc, or 293T cells (0.5 × 10⁷ to 2.0 × 10⁷ cells) that transiently expressed human FCRL1, cynomolgus monkey FCRL1 or mutants thereof were used.

One or more of these immunogens were injected intramuscularly, intradermally, intraperitoneally, or intravenously 3 times to 8 times at intervals of 10 days to 50 days based on various regimens. In the case of using an adjuvant, a Sigma adjuvant system (Sigma-Aldrich) was used. Mice were selected based on the reactivity evaluation of antiserum to various antigen-expressing cells, and 3 days after the final immunization, cell fusion of spleen cells and mouse myeloma cells P3U1 was performed to produce monoclonal antibody-producing hybridomas.

Among the obtained novel anti-FCRL1 mouse antibody, screening was performed based on an in vitro anti-cellular activity and a cross-reactivity to cynomolgus monkey FCRL1 using a second immunotoxin, and further, an antibody for ADC conversion was selected from the viewpoint of whether the amino acid sequence was suitable for antibody engineering modification for ADC conversion. VHs and VLs of the selected six antibodies (DK610, DK681, DK1142, DK1141, DK1166, and DK1164) and the amino acid sequences of heavy chain CDRs 1 to 3 (HCDRs 1 to 3) and light chain CDRs 1 to 3 (LCDRs 1 to 3) are shown in Table 2.

**[Table 2]**

| Clone name | VH | HCDR1 | HCDR2 | HCDR3 | VL | LCDR1 | LCDR2 | LCDR3 |
|---|---|---|---|---|---|---|---|---|
| DK610 | SEQ ID NO: 19 | SEQ ID NO: 20 | SEQ ID NO: 21 | SEQ ID NO: 22 | SEQ ID NO: 23 | SEQ ID NO: 24 | SEQ ID NO: 25 | SEQ ID NO: 26 |
| DK681 | SEQ ID NO: 27 | SEQ ID NO: 28 | SEQ ID NO: 29 | SEQ ID NO: 30 | SEQ ID NO: 31 | SEQ ID NO: 32 | SEQ ID NO: 33 | SEQ ID NO: 34 |
| DK1142 | SEQ ID NO: 35 | SEQ ID NO: 36 | SEQ ID NO: 37 | SEQ ID NO: 38 | SEQ ID NO: 39 | SEQ ID NO: 40 | SEQ ID NO: 41 | SEQ ID NO: 42 |
| DK1141 | SEQ ID NO: 43 | SEQ ID NO: 44 | SEQ ID NO: 45 | SEQ ID NO: 46 | SEQ ID NO: 47 | SEQ ID NO: 48 | SEQ ID NO: 49 | SEQ ID NO: 50 |
| DK1166 | SEQ ID NO: 51 | SEQ ID NO: 52 | SEQ ID NO: 53 | SEQ ID NO: 54 | SEQ ID NO: 55 | SEQ ID NO: 56 | SEQ ID NO: 57 | SEQ ID NO: 58 |
| DK1164 | SEQ ID NO: 59 | SEQ ID NO: 60 | SEQ ID NO: 61 | SEQ ID NO: 62 | SEQ ID NO: 63 | SEQ ID NO: 64 | SEQ ID NO: 65 | SEQ ID NO: 66 |

### [Example 5] FCRL 1 Binding Property of Novel Anti-human FCRL 1 Mouse Antibody

An antigen-specific reactivity of the novel anti-human FCRL1 mouse antibody obtained in Example 4 was evaluated by Flow Cytometry (FCM). FCRL1 antigen-expressing cells were prepared by transiently expressing the recombinant FCRL1 antigen on 293T cells. A codon-optimized cDNA was synthesized based on the amino acid sequence of human FCRL1 (NCBI accession number: NP_443170.1) or cynomolgus monkey FCRL1 (XP_015310712), and a fragment in which the sequence of an internal ribosome entry site (IRES) derived from the encephalomyocarditis virus (EMCV) and the sequence of TagBFP were arranged in tandem was cloned into pcDNA 3.1hygro to construct an expression plasmid vector for human FCRL1 or cynomolgus monkey FCRL1. The purified expression vector was transfected into 293T cells, cells were collected 2 days after transfection, and binding with each antibody was confirmed by FCM.

The FCM was performed under the following conditions. The collected cells were suspended with Dulbecco's PBS (FCM buffer) containing 5% FBS, 25% DMEM, and 0.1% sodium azide to a concentration of 2 × 10⁶ cells/mL. 25 µL of the cell suspension and 25 µL of each antibody solution diluted with PBS to 1 µg/mL were mixed in each well of a 96-well V plate (5 × 10⁴ cells/well, 500 ng/mL of each MAb), followed by reacting for 30 minutes at 4°C.

After centrifuging the plate and removing the supernatant, the plate was washed once by adding 200 µL/well of FCM buffer to suspend and re-centrifuging. The washed cells were re-suspended by adding 25 µL/well of FCM buffer diluted solution of a secondary antibody, followed by reacting at 4°C for 30 minutes.

As the secondary antibody, when the sample is a mouse antibody, R-Phycoerythrin (PE) F(ab')₂ Fragment Goat Anti-Mouse IgG (H+L) (Jackson ImmunoResearch) was used, and when the sample is a human antibody or a human chimeric antibody, R-Phycoerythrin (PE) F(ab')₂ Fragment Goat Anti-Human IgG, Fcy Fragment Specific (Jackson ImmunoResearch) was used. A solution of each secondary antibody was prepared by diluting the stock solution 200 times with an FCM buffer. After washing once, cells in each well were suspended with 100 µL of FCM buffer, and binding to each antibody was confirmed by flow cytometry (FCM).

A binding affinity of the antibody was determined by calculating a dissociation constant (K_{D}) as an apparent affinity for FCRL 1-expressing cells in FCM.

Cells that transiently expressed human FCRL 1 or cynomolgus monkey FCRL1 were reacted with a 3-fold dilution series of each antibody starting from 100 nM (15 µg/ml) at 12 points. Data obtained by the FCM was analyzed, and a binding amount of each antibody bound to the cell was calculated as a fluorescence amount MFI derived from the PE-labeled secondary antibody. An antibody concentration and the measured MFI were plotted with respect to a logarithm of the antibody concentration, a saturated binding curve of each antibody was obtained, and regression was performed by a 4-parameter logistic model. The dissociation constant (K_{D}) was calculated based on the obtained 50% effect concentration (EC₅₀) using Prism 5 software (Graph Pad Software Inc.) for fitting. The binding activity to human or cynomolgus monkey FCRL1 is shown in Table 3.

**[Table 3]**

| Clone name | DK610 | DK681 | DK1142 | DK1141 | DK1166 | DK1164 |
|---|---|---|---|---|---|---|
| Human FCRL1 (nM) | 0.4 | 1.0 | 1.4 | 1.6 | 1.5 | 2.3 |
| Human cynomolgus monkey FCRL1 (nM) | No binding | 0.8 | 1.1 | 0.8 | 1.0 | 1.4 |

### [Example 6] Preparation of ADC of Novel Anti-human FCRL 1 Chimeric Antibody

Based on amino acid sequence information on a variable region of the novel anti-human FCRL1 mouse antibody obtained in Example 4, a novel anti-FCRL1 antibody having S239C mutation and an ADC obtained by specifically adding SG3249 to a mutation site of the antibody were prepared according to the methods in Examples 1 and 2. Drug-to-antibody ratios of all ADCs were 1.8 to 1.9.

### [Example 7] Anti-cellular Test of ADC of Novel Anti-human FCRL 1 Chimeric Antibody

SU-DHL-6 cells were seeded in a 384-well plate (Greiner-Bio) at 40 µL/well so as to be 5000 cells/well. Ramos cells were seeded in a 96-well plate (Thermo Fisher Scientific) at 80 µL/well so as to be 4000 cells/well. A dilution ratio of ADC was set to V10 times, and 9 or 10 points were prepared with the highest concentration being 10000 ng/mL (final concentration: 10 ng/mL to 10000 ng/mL). An ADC diluted to a desired final concentration was added to a 384-well plate at 10 µL/well and to a 96-well plate at 20 µL/well.

After addition of ADC, cells were cultured in a carbon dioxide gas incubator set at 37°C for about 4 days. After completion of the culture, a Cell Titer-Glo Luminescent Cell Viability Assay (Promega) was added to a 384-well plate at 20 µL/well and to a 96-well plate at 100 µL/well, followed by reacting for about 15 minutes, and then live cells were measured by measuring luminescence values. The ADC prepared in Example 6 was used as the ADC of the novel anti-human FCRL1 chimeric antibody, and the ADC prepared in Example 2 was used as the 7G8-ADC.

Results of an anti-cell test for SU-DHL-6 cells are shown in FIGS. 2A and 2B, and results of an anti-cell test for Ramos cells are shown in FIGS. 3A and 3B. As shown in FIGS. 2A, 2B, 3A, and 3B, the ADCs of all novel anti-FCRL 1 antibodies have an anti-cellular effect on SU-DHL-6 cells and Ramos cells stronger than those of known anti-FCRL1 antibodies.

### [Example 8] Anti-tumor Test of ADC of Novel Anti-human FCRL 1 Chimeric Antibody

A Ramos cell subcutaneous grafted mouse model was prepared by the following method. Ramos cells were suspended in PBS, and subcutaneously grafted into a ventral side of SCID mice at 5 × 10⁶ cells/0.05 mL/head. On day 7 after grafting, individuals having a tumor volume of 85 mm³ or more were selected and grouped.

The Ramos cell subcutaneous grafted mouse model thus prepared and the SU-DHL-6 cell subcutaneous grafted mouse model prepared by the method in Example 3 were used in the following anti-tumor test.

On the grouping day (day 0), a diluted novel anti-FCRL1 chimeric antibody-ADC or 7G8-ADC of 0.3 mg/kg body weight was administered into the tail vein. A composition of a Vehicle is 10 mmol/L L-sodium glutamate, 262 mmol/L D-sorbitol, 0.05 mg/mL polysorbate 80, and pH 5.5. An average tumor volume of the 7G8-ADC administration group on Day 10 was taken as 1, and a relative value of an average tumor volume of each of novel anti-human FCRL1 chimeric antibody-ADC administration groups is shown in FIG. 4. The ADC prepared in Example 6 was used as the ADC of the novel anti-human FCRL1 chimeric antibody, and the ADC prepared in Example 2 was used as the 7G8-ADC.

As shown in FIG. 4, all the novel anti-human FCRL 1 chimeric antibody-ADCs had an anti-tumor activity stronger than that of 7G8-ADC. FIG. 5 shows a tumor size of the Ramos cell subcutaneous grafted mouse model on day 42. As shown in FIG. 5, it was revealed that while the 7G8-ADC was observed to cause tumor growth, all the ADCs of the novel anti-human FCRL1 chimeric antibody exhibited sustained and strong drug efficacy. These results showed that the novel anti-FCRL 1 antibody-ADC had an anti-tumor effect better than that of the known anti-FCRL 1 antibody-ADC.

### [Example 9] Internalization of Novel Anti-human FCRL1 Chimeric Antibody

The novel anti-human FCRL1 chimeric antibody produced in Example 6 was labeled with an IncuCyte Human Fab Fluor-pH Red Antibody Labeling Reagent (Sartorius) according to the attached instructions. Ramos cells were treated with a labeled antibody diluted to a final concentration of 200 ng/mL. After culturing in a carbon dioxide gas incubator set at 37°C for about 4 hours or 24 hours, a mean fluorescence intensity (MFI) was measured by FCM. The more internalized the antibody, the higher the MFI.

The internalization results are shown in FIG. 6. As shown in FIG. 6, all the novel anti-human FCRL1 chimeric antibodies had an internalization ability higher than that of the known anti-human FCRL 1 chimeric antibody.

### [Example 10] Preparation of Novel Anti-human FCRL 1 Humanized Antibody

### (1) Design of Amino Acid Sequences of VHs and VLs of DK681 Humanized Antibody and DK1142 Humanized Antibody

Amino acid sequences of various VHs and VLs of a DK681 humanized antibody and a DK1142 humanized antibody were designed by a method described below. In the following description, the DK681 humanized antibody and the DK1142 humanized antibody each comprising amino acid sequences of various VHs and VLs are collectively referred to as an hzDK681 antibody and an hzDK1142 antibody. As the amino acid sequence of a known human antibody framework (hereinafter, referred to as FR) suitable for grafting the amino acid sequences of CDRs of the variable regions of the DK681 mouse antibody and the DK1142 mouse antibody obtained in Example 4, the following were selected from search for a human FR consensus sequence reported by Kabat et al. [Sequences of Proteins of Immunological Interest, US Dept. Health and Human Services (1991)], a human antibody germline sequence, and a human antibody variable region-derived FR sequence obtained by a similarity search such as BLAST [J. Mol. Biol., 215, 403 (1990)]. For DK681, Genbank Accession Number: AKU38660.1 and Genbank Accession Number: AAW69164.1 were selected, and for DK1142, human subgroup H chain I (hereinafter, also referred to as hSGHI) and Genbank Accession Number: ABG38363.1 reported by Kabat et al. [Sequences of Proteins of Immunological Interest, US Dept. Health and Human Services (1991)] were selected, and the CDR was grafted into the FR of the same.

For DK681, amino acid sequences of CDRs 1 to 3 of a DK681 VH that are represented by SEQ ID NOs: 28, 29, and 30, respectively, were grafted into appropriate positions of an amino acid sequence of an FR of AKU38660.1 to design hzDK681 HV0 (SEQ ID NO: 67). Amino acid sequences of CDRs 1 to 3 of DK681 VL that are represented by SEQ ID NOs: 32, 33, and 34, respectively, were grafted into appropriate positions of an amino acid sequence of an FR of AAW69164.1 (an FR of a DK681 chimeric antibody was used as an FR4) to design hzDK681 LV0 (SEQ ID NO: 68).

By computer modeling of hzDK681 HV0 and hzDK681 LV0 designed as described above, an amino acid residue of an FR that is considered to affect an antibody binding activity was identified. As a result, a three-dimensional structure of an antigen binding site among amino acid residues of FRs of variable regions of hzDK681 HV0 and hzDK681 LV0 was changed, and as the amino acid residues considered to affect the binding activity of the antibody, Val at position 11, Lys at position 12, Arg at position 38, Met at position 48, Arg at position 67, Val at position 68, Ile at position 70, Ala at position 72, Thr at position 74, and Ala at position 97 in the amino acid sequence represented by SEQ ID NO: 67 were selected in the VH, and Ile at position 21, Pro at position 49, and Val at position 91 in the amino acid sequence represented by SEQ ID NO: 68 were selected in the VL. Among these selected amino acid residues, at least one amino acid residue was substituted with an amino acid residue present at the same site of the DK681 antibody, and the VH (SEQ ID NO: 72 and SEQ ID NO: 73) and the VL (SEQ ID NO: 74) of a humanized antibody comprising various modifications were designed.

For DK1142, amino acid sequences of CDRs 1 to 3 of DK1142 VH that are represented by SEQ ID NOs: 36, 37, and 38, respectively, were grafted into appropriate positions of an amino acid sequence of an FR of hSGHI to design hzDK1142 HV0 (SEQ ID NO: 69). Amino acid sequences of CDRs 1 to 3 of DK1142 VL that are represented by SEQ ID NOs: 40, 41, and 42, respectively, were grafted into appropriate positions of an amino acid sequence of an FR of ABG38363.1 (an FR of a DK1142 chimeric antibody was used as an FR4) to design hzDK1142 LV0 (SEQ ID NO: 70).

By computer modeling of hzDK1142 HV0 and hzDK1142 LV0 designed as described above, an amino acid residue of an FR that is considered to affect an antibody binding activity was identified. As a result, a three-dimensional structure of an antigen binding site among amino acid residues of FRs of variable regions of hzDK1142 HV0 and hzDK1142 LV0 was changed, and as the amino acid residues considered to affect the binding activity of the antibody, Val at position 11, Lys at position 12, Arg at position 38, Met at position 48, Arg at position 67, Val at position 68, lie at position 70, Thr at position 74, and Thy at position 95 in the amino acid sequence represented by SEQ ID NO: 69 were selected in the VH, and lie at position 2, Pro at position 15, and Gln at position 50 in the amino acid sequence represented by SEQ ID NO: 70 were selected in the VL. Among these selected amino acid residues, at least one amino acid residue was substituted with an amino acid residue present at the same site of the DK1142 antibody, and the VH (SEQ ID NO: 75 and SEQ ID NO: 77) and the VL (SEQ ID NO: 76) of a humanized antibody comprising various modifications were designed.

In addition to the standard CDR grafting design as described above, a VL CDR modification was also made to a part of a VL of the hzDK1142 antibody. Specifically, a CDR2 of a VL that is represented by SEQ ID NO: 71, in which Val at position 2 in the amino acid sequence of a CDR2 of a VL that is represented by SEQ ID NO: 41 was substituted with Ile, was introduced. A humanized antibody VL (SEQ ID NO: 78) was designed comprising the CDR2 of the VL that is represented by SEQ ID NO: 71.

### (2) Design of Variable Region Gene of Humanized Antibody

The DK681 humanized antibodies designed in this manner were named DK681 F11, DK681 F12, DK681 F13, and DK681 F14, respectively, and the DK1142 humanized antibodies were named DK1142 F21, DK1142 F22, and DK1142 F24, respectively. The variable region and CDRs of the humanized antibody are shown in Table 4. The nucleotide sequence encoding the amino acid sequence of the variable region of the humanized antibody was designed using codons frequently used in animal cells.

**[Table 4]**

| Clone name | VH | HCDR1 | HCDR2 | HCDR3 | VL | LCDR1 | LCDR2 | LCDR3 |
|---|---|---|---|---|---|---|---|---|
| DK681 F11 | SEQ ID NO: 72 | SEQ ID NO: 28 | SEQ ID NO: 29 | SEQ ID NO: 30 | SEQ ID NO: 68 | SEQ ID NO: 32 | SEQ ID NO: 33 | SEQ ID NO: 34 |
| DK681 F12 | SEQ ID NO: 73 | SEQ ID NO: 28 | SEQ ID NO: 29 | SEQ ID NO: 30 | SEQ ID NO: 74 | SEQ ID NO: 32 | SEQ ID NO: 33 | SEQ ID NO: 34 |
| DK681 F13 | SEQ ID NO: 72 | SEQ ID NO: 28 | SEQ ID NO: 29 | SEQ ID NO: 30 | SEQ ID NO: 74 | SEQ ID NO: 32 | SEQ ID NO: 33 | SEQ ID NO: 34 |
| DK681 F14 | SEQ ID NO: 73 | SEQ ID NO: 28 | SEQ ID NO: 29 | SEQ ID NO: 30 | SEQ ID NO: 68 | SEQ ID NO: 32 | SEQ ID NO: 33 | SEQ ID NO: 34 |
| DK1142 F21 | SEQ ID NO: 75 | SEQ ID NO: 36 | SEQ ID NO: 37 | SEQ ID NO: 38 | SEQ ID NO: 76 | SEQ ID NO: 40 | SEQ ID NO: 41 | SEQ ID NO: 42 |
| DK1142 F22 | SEQ ID NO: 77 | SEQ ID NO: 36 | SEQ ID NO: 37 | SEQ ID NO: 38 | SEQ ID NO: 76 | SEQ ID NO: 40 | SEQ ID NO: 41 | SEQ ID NO: 42 |
| DK1142 F24 | SEQ ID NO: 77 | SEQ ID NO: 36 | SEQ ID NO: 37 | SEQ ID NO: 38 | SEQ ID NO: 78 | SEQ ID NO: 40 | SEQ ID NO: 71 | SEQ ID NO: 42 |

### (3) Preparation of Humanized Antibody

A gene fragment corresponding to the nucleotide sequence designed in (2) was introduced into an expression vector by seamless cloning to prepare a necessary plasmid. ApCI-OtCMV_hK vector comprising a signal sequence and a human κ chain constant region sequence was used as a VL expression vector, and a pCI-OtCAG_hG1 (S239C) vector comprising a signal sequence and a human γ chain constant region sequence was used as a VH expression vector. The constant region sequence of the pCI-OtCAG_hG1 (S239C) vector is a heavy chain constant region obtained by introducing the S239C mutation into human IgG1. The vectors were prepared by total synthesis using, as a common skeleton, a pCI vector manufactured by Promega and introducing a restriction enzyme site necessary for expressing human antibody genes. The completed plasmid was prepared in large amount using a QIAGEN Plasmid Plus Maxi kit (QIAGEN). Next, the target humanized antibody was transiently expressed using an Expi293 Expression System Kit (Thermo Fisher Scientific). The method for introducing the plasmid was in accordance with the attached document.

The light chain expression vector and the heavy chain expression vector were mixed at a ratio of 2:1 and introduced. Cells into which the plasmid was introduced were cultured under conditions of 37°C, 8% CO₂, and 125 rpm for 2 days to 4 days. Thereafter, a cell culture suspension was centrifuged, and the culture supernatant was collected through a 0.2 µm filter. A purified antibody was obtained from the culture supernatant by affinity purification using Mab Select SuRe (Cytiva).

Specifically, after equilibrating a resin filled in a column with PBS, the culture supernatant was added to the column, followed by washing with PBS, and the antibody was eluted using an elution buffer (20 mM citric acid, 50 mM NaCl, pH 3.4). The obtained antibody solution was neutralized by adding 1/10 amount of neutralization buffer (1 M phosphoric acid-NaOH, pH 7.0), and the solvent of the antibody solution was replaced with PBS using NAP25 (manufactured by Cytiva). The antibody solution after the buffer replacement was concentrated by ultrafiltration using Amicon Ultra-4 Centrifugal Filter Units (Millipore), and an absorbance A280 was measured using Nanodrop (Thermo Fisher Scientific), and a concentration of the antibody solution was measured and prepared. An absorption coefficient was calculated based on the amino acid sequence of each humanized antibody according to a method by C. N. Pace et al (1995, Prot. Sci.4: 2411-2423). The purified antibody was subjected to quality confirmation by gel filtration chromatography for analysis (using a column TSKgel Super SW 3000 manufactured by TOSOH CORPORATION) and SDS-PAGE.

### [Example 11] F CRL1 Binding Property of Novel Anti-human F CRL1 Humanized Antibody

For the purpose of comparing the binding activity of the DK681 chimeric antibody (chDK681) and the DK1 142 chimeric antibody (chDK1142) obtained by connecting constant regions of the DK681 and DK1142 mouse antibodies obtained in Example 4 to a human IgG1 (S239C) constant region with the binding activity of the anti-FCRL1 humanized antibody obtained in Example 10 to human FCRL1, the binding activity to hFCRL1/FcRH1-His (manufactured by R&D Systems) was measured by a surface plasmon resonance method (SPR method) using Biacore8K+ (manufactured by Cytiva).

The binding activity of the anti-FCRL 1 antibody was measured as follows. An anti-human IgG antibody was immobilized on a CM5 sensor chip (manufactured by Cytiva) in accordance with the attached protocol using a Human Antibody Capture Kit (manufactured by Cytiva). An anti-FCRL 1 antibody prepared at 5 µg/mL was added to a flow cell on which the Anti-human IgG antibody was immobilized at a flow rate of 10 µL/min for 30 seconds.

Next, hFCRL1/FcRH1-His, which is prepared stepwise to 5 concentrations by 3-fold dilution from 10000 ng/mL, was monitored at a flow rate of 30 µL/min for a binding reaction for 180 seconds and a dissociation reaction for 400 seconds. For the obtained sensorgram, a kinetic constant of each antibody was calculated by fitting by a Steady state affinity model or a 1:1 Binding model using Bia Evaluation Software (manufactured by Cytiva). The calculated binding rate constant (ka), dissociation rate constant (kd) and dissociation constant [KD] of each antibody are shown in Table 5. The chDK681 is referred to as DK681 F01, and the chDK1142 is referred to as DK1142 F02.

**[Table 5]**

| Drug Name | ka (1/Ms) | kd (1/s) | KD (M) | fitting model |
|---|---|---|---|---|
| DK681 F01 | 4.47 × 10⁵ | 1.05 × 10⁻³ | 6.65 × 10⁻⁹ | Steady state affinity model |
| DK681 F11 | 4.41 × 10⁵ | 6.06 × 10⁻³ | 1.76 × 10⁻⁸ | Steady state affinity model |
| DK681 F12 | 3.85 × 10⁵ | 2.59 × 10⁻³ | 9.15 × 10⁻⁹ | Steady state affinity model |
| DK681 F13 | 3.87 × 10⁵ | 2.97 × 10⁻³ | 9.84 × 10⁻⁹ | Steady state affinity model |
| DK681 F14 | 4.01 × 10⁵ | 5.08 × 10⁻³ | 1.61 × 10⁻⁸ | Steady state affinity model |
| DK1142 F02 | 4.55 × 10⁶ | 5.18 × 10⁻² | 1.14 × 10⁻⁸ | 1:1 Binding model |
| DK1142 F21 | 5.36 × 10⁶ | 6.86 × 10⁻² | 1.28 × 10⁻⁸ | 1:1 Binding model |
| DK1142 F22 | 3.85 × 10⁶ | 4.28 × 10⁻² | 1.11 × 10⁻⁸ | 1:1 Binding model |
| DK1142 F24 | 2.24 × 10⁶ | 2.74 × 10⁻² | 1.22 × 10⁻⁸ | 1:1 Binding model |

From the above results, it was revealed that the anti-human FCRL1 humanized antibody produced in Example 10 had a binding activity equivalent to those of the chDK681 antibody and the chDK1142 antibody.

### [Example 12] Preparation of ADC of Novel Anti-human FCRL1 Humanized Antibody

According to the methods in Examples 1 and 2, novel anti-FCRL 1 antibodies each comprising the S239C mutation and ADCs obtained by specifically adding SG3249 to mutation sites of the antibodies were produced. Sequences of variable regions of the novel anti-human FCRL 1 humanized antibodies were as shown in Table 4. Drug-to-antibody ratios of all ADCs were 1.7 to 1.8.

### [Example 13] Anti-cellular Test of ADC of Novel Anti-human FCRL 1 Humanized Antibody

According to the method in Example 7, an anti-cellular effect of the ADC of the novel anti-FCRL1 humanized antibody produced in Example 12 on SU-DHL-6 cells and Ramos cells was confirmed.

Results of an anti-cellular test for SU-DHL-6 cells are shown in FIGS. 7A and 7B, and results of an anti-cellular test for Ramos cells are shown in FIGS. 8A and 8B. As shown in FIGS. 7A, 7B, 8A, and 8B, the ADCs of all novel anti-FCRL 1 antibodies had an anti-cellular effect on SU-DHL-6 cells and Ramos cells stronger than that of the ADCs of known anti-FCRL1 antibodies.

### [Example 14] Anti-tumor Test of ADC of Novel Anti-human FCRL 1 Humanized Antibody

According to the methods in Examples 3 and 8, the produced SU-DHL-6 cell subcutaneous grafted mouse model and Ramos cell subcutaneous grafted mouse model were used in the following anti-tumor test.

On the grouping day (day 0), a diluted novel anti-human FCRL1 humanized antibody-ADC or 7G8-ADC of 0.3 mg/kg body weight was administered into the tail vein. A composition of a Vehicle is 10 mmol/L L-sodium glutamate, 262 mmol/L D-sorbitol, 0.05 mg/mL polysorbate 80, and pH 5.5. An average tumor volume of the 7G8-ADC administration group on Day 7 was taken as 1, and a relative value of an average tumor volume of each of novel anti-human FCRL1 humanized antibody-ADC administration groups is shown in FIG. 9. The ADC prepared in Example 12 was used as the ADC of the novel anti-human FCRL1 humanized antibody, and the ADC prepared in Example 2 was used as the 7G8-ADC.

As shown in FIG. 9, all the novel anti-FCRL1 humanized antibody-ADCs had an anti-tumor activity stronger than that of 7G8-ADC. These results showed that the novel anti-FCRL1 humanized antibody-ADC had an anti-tumor effect better than that of the known anti-FCRL 1 antibody-ADC.

### INDUSTRIAL APPLICABILITY

The present invention provides a novel monoclonal antibody or an antibody fragment thereof that binds to an extracellular region of FCRL 1.

Although the present invention has been described in detail with reference to specific aspects, it is obvious to those skilled in the art that various changes and modifications can be made without departing from the spirit and scope of the present invention. The present application is based on a Japanese patent application (JP2022-019051) filed on February 9, 2022, the disclosure of which is hereby incorporated by reference herein in its entirety. All references cited herein are incorporated in their entirety.

### SEQUENCE LISTING FREE TEXT

SEQ ID NO: 1: gene sequence of human FCRL 1
SEQ ID NO: 2: gene sequence of monkey FCRL 1
SEQ ID NO: 3: amino acid sequence of human FCRL1
SEQ ID NO: 4: amino acid sequence of monkey FCRL 1
SEQ ID NO: 5: amino acid sequence of VH of E3
SEQ ID NO: 6: amino acid sequence of VL of E3
SEQ ID NO: 7: amino acid sequence of VH of E9
SEQ ID NO: 8: amino acid sequence of VL of E9
SEQ ID NO: 9: amino acid sequence of VH of 1F9
SEQ ID NO: 10: amino acid sequence of VL of 1F9
SEQ ID NO: 11: amino acid sequence of VH of 2A10
SEQ ID NO: 12: amino acid sequence of VL of 2A10
SEQ ID NO: 13: amino acid sequence of VH of 7G8
SEQ ID NO: 14: amino acid sequence of VL of 7G8
SEQ ID NO: 15: amino acid sequence of VH of 2G5
SEQ ID NO: 16: amino acid sequence of VL of 2G5
SEQ ID NO: 17: amino acid sequence of VH of 5A2
SEQ ID NO: 18: amino acid sequence of VL of 5A2
SEQ ID NO: 19: amino acid sequence of VH of DK610
SEQ ID NO: 20: amino acid sequence of HCDR1 of DK610
SEQ ID NO: 21: amino acid sequence of HCDR2 of DK610
SEQ ID NO: 22: amino acid sequence of HCDR3 of DK610
SEQ ID NO: 23: amino acid sequence of DK610
SEQ ID NO: 24: amino acid sequence of LCDR1 of DK610
SEQ ID NO: 25: amino acid sequence of LCDR2 of DK610
SEQ ID NO: 26: amino acid sequence of LCDR3 of DK610
SEQ ID NO: 27: amino acid sequence of VH of DK681
SEQ ID NO: 28: amino acid sequence of HCDR1 of DK681
SEQ ID NO: 29: amino acid sequence of HCDR2 of DK681
SEQ ID NO: 30: amino acid sequence of HCDR3 of DK681
SEQ ID NO: 31: amino acid sequence of DK681
SEQ ID NO: 32: amino acid sequence of LCDR1 of DK681
SEQ ID NO: 33: amino acid sequence of LCDR2 of DK681
SEQ ID NO: 34: amino acid sequence of LCDR3 of DK681
SEQ ID NO: 35: amino acid sequence of VH of DK1142
SEQ ID NO: 36: amino acid sequence of HCDR1 of DK1142
SEQ ID NO: 37: amino acid sequence of HCDR2 of DK1142
SEQ ID NO: 38: amino acid sequence of HCDR3 of DK1142
SEQ ID NO: 39: amino acid sequence of DK1142
SEQ ID NO: 40: amino acid sequence of LCDR1 of DK1142
SEQ ID NO: 41: amino acid sequence of LCDR2 of DK1142
SEQ ID NO: 42: amino acid sequence of LCDR3 of DK1142
SEQ ID NO: 43: amino acid sequence of VH of DK1141
SEQ ID NO: 44: amino acid sequence of HCDR1 of DK1141
SEQ ID NO: 45: amino acid sequence of HCDR2 of DK1141
SEQ ID NO: 46: amino acid sequence of HCDR3 of DK1141
SEQ ID NO: 47: amino acid sequence of DK1141
SEQ ID NO: 48: amino acid sequence of LCDR1 of DK1141
SEQ ID NO: 49: amino acid sequence of LCDR2 of DK1141
SEQ ID NO: 50: amino acid sequence of LCDR3 of DK1141
SEQ ID NO: 51: amino acid sequence of VH of DK1166
SEQ ID NO: 52: amino acid sequence of HCDR1 of DK1166
SEQ ID NO: 53: amino acid sequence of HCDR2 of DK1166
SEQ ID NO: 54: amino acid sequence of HCDR3 of DK1166
SEQ ID NO: 55: amino acid sequence of DK1166
SEQ ID NO: 56: amino acid sequence of LCDR1 of DK1166
SEQ ID NO: 57: amino acid sequence of LCDR2 of DK1166
SEQ ID NO: 58: amino acid sequence of LCDR3 of DK1166
SEQ ID NO: 59: amino acid sequence of VH of DK1164
SEQ ID NO: 60: amino acid sequence of HCDR1 of DK1164
SEQ ID NO: 61: amino acid sequence of HCDR2 of DK1164
SEQ ID NO: 62: amino acid sequence of HCDR3 of DK1164
SEQ ID NO: 63: amino acid sequence of DK1164
SEQ ID NO: 64: amino acid sequence of LCDR1 of DK1164
SEQ ID NO: 65: amino acid sequence of LCDR2 of DK1164
SEQ ID NO: 66: amino acid sequence of LCDR3 of DK1164
SEQ ID NO: 67: amino acid sequence of hzDK681 HV0
SEQ ID NO: 68: amino acid sequence of hzDK681 LV0, and amino acid sequence of DK681 F11 and DK681 F14
SEQ ID NO: 69: amino acid sequence of hzDK1142 HV0
SEQ ID NO: 70: amino acid sequence of hzDK1142 LV0
SEQ ID NO: 71: amino acid sequence of LCDR2 of DK1142 F24
SEQ ID NO: 72: amino acid sequence of VH of DK681 F11 and DK681 F13
SEQ ID NO: 73: amino acid sequence of VH of DK681 F12 and DK681 F14
SEQ ID NO: 74: amino acid sequence of VL of DK681 F12 and DK681 F13
SEQ ID NO: 75: amino acid sequence of VH of DK1142 F21
SEQ ID NO: 76: amino acid sequence of VL of DK1142 F21 and DK1142 F22
SEQ ID NO: 77: amino acid sequence of VH of DK1142 F22 and DK1142 F24
SEQ ID NO: 78: amino acid sequence of VL of DK1142 F24
SEQ ID NO: 79: amino acid sequence of CH of human IgG1
SEQ ID NO: 80: amino acid sequence of CH of IgG1 (S239C)

## Claims

1. An antibody or an antibody fragment thereof, which is a monoclonal antibody or an antibody fragment thereof that binds to Fc receptor-like protein 1 (hereinafter, abbreviated as FCRL 1), wherein
the antibody is any one antibody selected from the following (a) to (g):
(a) an antibody in which complementarity determining regions (hereinafter, abbreviated as CDR) 1 to 3 of a heavy chain variable region (hereinafter, abbreviated as VH) comprise the amino acid sequences represented by SEQ ID NOs: 20 to 22, respectively, and CDRs 1 to 3 of a light chain variable region (hereinafter, abbreviated as VL) comprise the amino acid sequences represented by SEQ ID NOs: 24 to 26, respectively,
(b) an antibody in which CDRs 1 to 3 of a VH comprise the amino acid sequences represented by SEQ ID NOs: 28 to 30, respectively, and CDRs 1 to 3 of a VL comprise the amino acid sequences represented by SEQ ID NOs: 32 to 34, respectively,
(c) an antibody in which CDRs 1 to 3 of a VH comprise the amino acid sequences represented by SEQ ID NOs: 36 to 38, respectively, and CDRs 1 to 3 of a VL comprise the amino acid sequences represented by SEQ ID NOs: 40 to 42, respectively,
(d) an antibody in which CDRs 1 to 3 of a VH comprise the amino acid sequences represented by SEQ ID NOs: 44 to 46, respectively, and CDRs 1 to 3 of a VL comprise the amino acid sequences represented by SEQ ID NOs: 48 to 50, respectively,
(e) an antibody in which CDRs 1 to 3 of a VH comprise the amino acid sequences represented by SEQ ID NOs: 52 to 54, respectively, and CDRs 1 to 3 of a VL comprise the amino acid sequences represented by SEQ ID NOs: 56 to 58, respectively, and
(f) an antibody in which CDRs 1 to 3 of a VH comprise the amino acid sequences represented by SEQ ID NOs: 60 to 62, respectively, and CDRs 1 to 3 of a VL comprise the amino acid sequences represented by SEQ ID NOs: 64 to 66, respectively, and
(g) an antibody in which CDRs 1 to 3 of a VH comprise the amino acid sequences represented by SEQ ID NOs: 36 to 38, respectively, and CDRs 1 to 3 of a VL comprise the amino acid sequences represented by SEQ ID NOs: 40, 71, and 42, respectively.

2. An antibody or an antibody fragment thereof, which is a monoclonal antibody or an antibody fragment thereof that binds to FCRL 1, wherein
the antibody is any one antibody selected from the following (2b-1) to (2b-4), (2c-1), (2c-2), and (2g-1):
(2b-1) an antibody in which a VH comprises the amino acid sequence represented by SEQ ID NO: 72 and a VL comprises the amino acid sequence represented by SEQ ID NO: 68,
(2b-2) an antibody in which a VH comprises the amino acid sequence represented by SEQ ID NO: 73 and a VL comprises the amino acid sequence represented by SEQ ID NO: 74,
(2b-3) an antibody in which a VH comprises the amino acid sequence represented by SEQ ID NO: 72 and a VL comprises the amino acid sequence represented by SEQ ID NO: 74,
(2b-4) an antibody in which a VH comprises the amino acid sequence represented by SEQ ID NO: 73 and a VL comprises the amino acid sequence represented by SEQ ID NO: 68,
(2c-1) an antibody in which a VH comprises the amino acid sequence represented by SEQ ID NO: 75 and a VL comprises the amino acid sequence represented by SEQ ID NO: 76,
(2c-2) an antibody in which a VH comprises the amino acid sequence represented by SEQ ID NO: 77 and a VL comprises the amino acid sequence represented by SEQ ID NO: 76, and
(2g-1) an antibody in which a VH comprises the amino acid sequence represented by SEQ ID NO: 77 and a VL comprises the amino acid sequence represented by SEQ ID NO: 78.

3. The antibody or the antibody fragment thereof according to claim 1 or 2, wherein
a heavy chain constant region of the antibody is a heavy chain constant region of IgG.

4. The antibody or the antibody fragment thereof according to claim 3, wherein
the heavy chain constant region of the antibody comprises the amino acid sequence represented by SEQ ID NO: 79 or 80.

5. The antibody or the antibody fragment thereof according to any one of claims 1 to 4, wherein
the antibody is a genetically recombinant antibody.

6. The antibody or the antibody fragment thereof according to claim 5, wherein
the genetically recombinant antibody is one selected from the group consisting of a chimeric antibody, a humanized antibody, and a human antibody.

7. The antibody fragment according to any one of claims 1 to 6, wherein
the antibody fragment is one selected from Fab, Fab', F(ab')₂, a single chain antibody (scFv), a dimerized V region (diabody), a disulfide-stabilized V region (dsFv), and a peptide comprising a CDR.

8. A hybridoma that produces the antibody according to any one of claims 1 to 6.

9. A nucleic acid comprising a nucleotide sequence that encodes the antibody or the antibody fragment thereof according to any one of claims 1 to 7.

10. A vector comprising the nucleic acid according to claim 9.

11. A transformed cell obtained by introducing the vector according to claim 10 into a host cell.

12. A method for producing the antibody or the antibody fragment thereof according to any one of claims 1 to 7, comprising:
culturing the hybridoma according to claim 8 or the transformed cell according to claim 11 in a medium, and collecting the antibody or the antibody fragment thereof from a culture.

13. An antibody-drug conjugate comprising:
the antibody or the antibody fragment thereof according to any one of claims 1 to 7.

14. The antibody-drug conjugate according to claim 13, wherein
the antibody-drug conjugate comprises the antibody or the antibody fragment thereof linked to a drug via a linker.

15. A composition comprising:
the antibody or the antibody fragment thereof according to any one of claims 1 to 7, or the antibody-drug conjugate according to claim 13 or 14.

16. A reagent for detecting or measuring FCRL 1, comprising:
the antibody or the antibody fragment thereof according to any one of claims 1 to 7, or the antibody-drug conjugate according to claim 13 or 14.

17. A diagnostic agent for a disease associated with FCRL 1, comprising:
the antibody or the antibody fragment thereof according to any one of claims 1 to 7, or the antibody-drug conjugate according to claim 13 or 14.

18. The diagnostic agent according to claim 17, wherein
the disease associated with FCRL 1 is cancer, an autoimmune disease or an inflammatory disease.

19. A therapeutic agent for a disease associated with FCRL 1, comprising:
the antibody or the antibody fragment thereof according to any one of claims 1 to 7, or the antibody-drug conjugate according to claim 13 or 14.

20. The therapeutic agent according to claim 19, wherein
the disease associated with FCRL 1 is cancer, an autoimmune disease, or an inflammatory disease.

21. A diagnostic method for a disease associated with FCRL 1, comprising:
using the antibody or the antibody fragment thereof according to any one of claims 1 to 7, or the antibody-drug conjugate according to claim 13 or 14.

22. A therapeutic method for a disease associated with FCRL 1, comprising:
administering the antibody or the antibody fragment thereof according to any one of claims 1 to 7, or the antibody-drug conjugate according to claim 13 or 14.

23. Use of the antibody or the antibody fragment thereof according to any one of claims 1 to 7, or the antibody-drug conjugate according to claim 13 or 14, for manufacturing a diagnostic agent for a disease associated with FCRL1.

24. Use of the antibody or the antibody fragment thereof according to any one of claims 1 to 7, or the antibody-drug conjugate according to claim 13 or 14, for manufacturing a therapeutic agent for a disease associated with FCRL1.

25. Use of the antibody or the antibody fragment thereof according to any one of claims 1 to 7, or the antibody-drug conjugate according to claim 13 or 14, for use as a diagnostic agent for a disease associated with FCRL 1.

26. Use of the antibody or the antibody fragment thereof according to any one of claims 1 to 7, or the antibody-drug conjugate according to claim 13 or 14, for use as a therapeutic agent for a disease associated with FCRL 1.
